(19) Europäisches Patentamt
European Patent Office
Office européen des brevets

(11) EP 0 840 803 B1

(12) EUROPEAN PATENT SPECIFICATION

(45) Date of publication and mention of the grant of the patent:
**02.05.2003 Bulletin 2003/18**

(21) Application number: **96942790.5**

(22) Date of filing: **19.11.1996**

(51) Int Cl.[7]: **C12Q 1/68**

(86) International application number:
**PCT/US96/18708**

(87) International publication number:
**WO 97/032999 (12.09.1997 Gazette 1997/39)**

(54) **SIMULTANEOUS SEQUENCING OF TAGGED POLYNUCLEOTIDES**

GLEICHZEITIGE SEQUENZIERUNG VON MARKIERTEN POLYNUKLEOTIDEN

SEQUENCAGE SIMULTANE DE POLYNUCLEOTIDES MARQUES

(84) Designated Contracting States:
**BE CH DE DK FR GB IT LI NL SE**

(30) Priority: **05.03.1996 US 611155**

(43) Date of publication of application:
**13.05.1998 Bulletin 1998/20**

(73) Proprietor: **LYNX THERAPEUTICS, INC.**
**Hayward, CA 94545 (US)**

(72) Inventor: **BRENNER, Sydney**
**Cambridge, CB2 3PJ (GB)**

(74) Representative: **VOSSIUS & PARTNER**
**Postfach 86 07 67**
**81634 München (DE)**

(56) References cited:

| | |
|---|---|
| **EP-A- 0 303 459** | **EP-A- 0 630 972** |
| **EP-A- 0 649 852** | **EP-A- 0 701 001** |
| **WO-A-93/17126** | **WO-A-95/04160** |
| **WO-A-95/27080** | **WO-A-96/12014** |
| **WO-A-96/12039** | **WO-A-96/41011** |

**EP 0 840 803 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

SEQUENCE LISTING

(1) GENERAL INFORMATION:

(i) APPLICANT: Sydney Brenner

(ii) TITLE OF INVENTION: Simultaneous Sequencing of Tagged Polynucleotides

(iii) NUMBER OF SEQUENCES: 27

(iv) CORRESPONDENCE ADDRESS:

(A) ADDRESSEE: Stephen C. Macevicz, Lynx Therapeutics, Inc.
(B) STREET: 3832 Bay Center Place
(C) CITY: Hayward
(D) STATE: California
(E) COUNTRY: USA
(F) ZIP: 94545

(v) COMPUTER READABLE FORM:

(A) MEDIUM TYPE: 3.5 inch diskette
(B) COMPUTER: IBM compatible
(C) OPERATING SYSTEM: Windows 3.1
(D) SOFTWARE: Microsoft Word ver. 5.1

(vi) CURRENT APPLICATION DATA:

(A) APPLICATION NUMBER:
(B) FILING DATE:
(C) CLASSIFICATION:

(vii) PRIOR APPLICATION DATA:

(A) APPLICATION NUMBER: 08/560,313
(B) FILING DATE: 17-NOV-95

(vii) PRIOR APPLICATION DATA:

(A) APPLICATION NUMBER: 08/611,155
(B) FILING DATE: 05-MAR-96

(viii) ATTORNEY/AGENT INFORMATION:

(A) NAME: Stephen C. Macevicz
(B) REGISTRATION NUMBER: 30,285
(C) REFERENCE/DOCKET NUMBER: 807wo

(ix) TELECOMMUNICATION INFORMATION:

(A) TELEPHONE: (510) 670-9365
(B) TELEFAX: (510) 670-9302

(2) INFORMATION FOR SEQ ID NO: 1:

(i) SEQUENCE CHARACTERISTICS:

(A) LENGTH: 22 nucleotides
(B) TYPE: nucleic acid
(C) STRANDEDNESS: single
(D) TOPOLOGY: linear

(xi) SEQUENCE DESCRIPTION: SEQ ID NO: 1:

GGAAGAGGAA GAGGAAGAYY YN 22

(2) INFORMATION FOR SEQ ID NO: 2:

(i) SEQUENCE CHARACTERISTICS:

(A) LENGTH: 22 nucleotides
(B) TYPE: nucleic acid
(C) STRANDEDNESS: single
(D) TOPOLOGY: linear

(xi) SEQUENCE DESCRIPTION: SEQ ID NO: 2:

GAAGAGGAAG AGGAAGAGYY YN 22

(2) INFORMATION FOR SEQ ID NO: 3:

(i) SEQUENCE CHARACTERISTICS:

(A) LENGTH: 22 nucleotides
(B) TYPE: nucleic acid
(C) STRANDEDNESS: single
(D) TOPOLOGY: linear

(xi) SEQUENCE DESCRIPTION: SEQ ID NO: 3:

AAGAGGAAGA GGAAGAGGYY YN 22

(2) INFORMATION FOR SEQ ID NO: 4:

(i) SEQUENCE CHARACTERISTICS:

(A) LENGTH: 22 nucleotides
(B) TYPE: nucleic acid
(C) STRANDEDNESS: single
(D) TOPOLOGY: linear

(xi) SEQUENCE DESCRIPTION: SEQ ID NO: 4:

AGAGGAAGAG GAAGAGGAYY YN 22

(2) INFORMATION FOR SEQ ID NO: 5:

(i) SEQUENCE CHARACTERISTICS:

(A) LENGTH: 22 nucleotides
(B) TYPE: nucleic acid
(C) STRANDEDNESS: single

(D) TOPOLOGY: linear

(xi) SEQUENCE DESCRIPTION: SEQ ID NO: 5:

GAGGAAGAGG AAGAGGAAYY YN 22

(2) INFORMATION FOR SEQ ID NO: 6:

(i) SEQUENCE CHARACTERISTICS:

(A) LENGTH: 22 nucleotides
(B) TYPE: nucleic acid
(C) STRANDEDNESS: single
(D) TOPOLOGY: linear

(xi) SEQUENCE DESCRIPTION: SEQ ID NO: 6:

AGGAAGAGGA AGAGGAAGYY YN 22

(2) INFORMATION FOR SEQ ID NO: 7:

(i) SEQUENCE CHARACTERISTICS:

(A) LENGTH: 19 nucleotides
(B) TYPE: nucleic acid
(C) STRANDEDNESS: double
(D) TOPOLOGY: linear

(xi) SEQUENCE DESCRIPTION: SEQ ID NO: 7:

AAAGAAAGGA AGGGCAGCT 19

(2) INFORMATION FOR SEQ ID NO: 8:

(i) SEQUENCE CHARACTERISTICS:

(A) LENGTH: 16 nucleotides
(B) TYPE: nucleic acid
(C) STRANDEDNESS: double
(D) TOPOLOGY: linear

(xi) SEQUENCE DESCRIPTION: SEQ ID NO: 8:

NNGGATGNNN NNNNNN 16

(2) INFORMATION FOR SEQ ID NO: 9:

(i) SEQUENCE CHARACTERISTICS:

(A) LENGTH: 11 nucleotides

(B) TYPE: nucleic acid
(C) STRANDEDNESS: double
(D) TOPOLOGY: linear

(xi) SEQUENCE DESCRIPTION: SEQ ID NO: 9:

ACCAGCTGAT C 11

(2) INFORMATION FOR SEQ ID NO: 10:

(i) SEQUENCE CHARACTERISTICS:

(A) LENGTH: 11 nucleotides
(B) TYPE: nucleic acid
(C) STRANDEDNESS: double
(D) TOPOLOGY: linear

(xi) SEQUENCE DESCRIPTION: SEQ ID NO: 10:

NRRGATCYNN N 11

(2) INFORMATION FOR SEQ ID NO: 11:

(i) SEQUENCE CHARACTERISTICS:

(A) LENGTH: 48 nucleotides
(B) TYPE: nucleic acid
(C) STRANDEDNESS: single
(D) TOPOLOGY: linear

(xi) SEQUENCE DESCRIPTION: SEQ ID NO: 11:

GAGGATGCCT TTATGGATCC ACTCGAGATC CCAATCCA 48

(2) INFORMATION FOR SEQ ID NO: 12:

(i) SEQUENCE CHARACTERISTICS:

(A) LENGTH: 20 nucleotides
(B) TYPE: nucleic acid
(C) STRANDEDNESS: double
(D) TOPOLOGY: linear

(xi) SEQUENCE DESCRIPTION: SEQ ID NO: 12:

AGTGGCTGGG CATCGGACCG 20

(2) INFORMATION FOR SEQ ID NO: 13:

(i) SEQUENCE CHARACTERISTICS:

(A) LENGTH: 20 nucleotides
(B) TYPE: nucleic acid
(C) STRANDEDNESS: double
(D) TOPOLOGY: linear

(xi) SEQUENCE DESCRIPTION: SEQ ID NO: 13:

GGGGCCCAGT CAGCGTCGAT 20

(2) INFORMATION FOR SEQ ID NO: 14:

(i) SEQUENCE CHARACTERISTICS:

(A) LENGTH: 62 nucleotides
(B) TYPE: nucleic acid
(C) STRANDEDNESS: double
(D) TOPOLOGY: linear

(xi) SEQUENCE DESCRIPTION: SEQ ID NO: 14:

AAAAGGAGGA GGCCTTGATA GAGAGGACCT GTTTAAACGG ATCCGCTGCT 50
TTTTCCTCCT CC 62

(2) INFORMATION FOR SEQ ID NO: 15:

(i) SEQUENCE CHARACTERISTICS:

(A) LENGTH: 26 nucleotides
(B) TYPE: nucleic acid
(C) STRANDEDNESS: single
(D) TOPOLOGY: linear

(xi) SEQUENCE DESCRIPTION: SEQ ID NO: 15:

NNNNNNNNAA AAGGAGGAGG CCTTGA 26

(2) INFORMATION FOR SEQ ID NO: 16:

(i) SEQUENCE CHARACTERISTICS:

(A) LENGTH: 26 nucleotides
(B) TYPE: nucleic acid
(C) STRANDEDNESS: double
(D) TOPOLOGY: linear

(xi) SEQUENCE DESCRIPTION: SEQ ID NO: 16:

NNNNNNNGGA GGAGGAAAAA GCAGCN 26

(2) INFORMATION FOR SEQ ID NO: 17:

(i) SEQUENCE CHARACTERISTICS:

(A) LENGTH: 26 nucleotides
(B) TYPE: nucleic acid
(C) STRANDEDNESS: double
(D) TOPOLOGY: linear

(xi) SEQUENCE DESCRIPTION: SEQ ID NO: 17:

NNNNNNGGAG GAGGAAAAAG CAGCNN 26

(2) INFORMATION FOR SEQ ID NO: 18:

(i) SEQUENCE CHARACTERISTICS:

(A) LENGTH: 26 nucleotides
(B) TYPE: nucleic acid
(C) STRANDEDNESS: double
(D) TOPOLOGY: linear

(xi) SEQUENCE DESCRIPTION: SEQ ID NO: 18:

NNNNNGGAGG AGGAAAAAGC AGCNNN 26

(2) INFORMATION FOR SEQ ID NO: 19:

(i) SEQUENCE CHARACTERISTICS:

(A) LENGTH: 26 nucleotides
(B) TYPE: nucleic acid
(C) STRANDEDNESS: double
(D) TOPOLOGY: linear

(xi) SEQUENCE DESCRIPTION: SEQ ID NO: 19:

NNNNGGAGGA GGAAAAAGCA GCNNNN 26

(2) INFORMATION FOR SEQ ID NO: 20:

(i) SEQUENCE CHARACTERISTICS:

(A) LENGTH: 26 nucleotides
(B) TYPE: nucleic acid
(C) STRANDEDNESS: double
(D) TOPOLOGY: linear

(xi) SEQUENCE DESCRIPTION: SEQ ID NO: 20:

NNNGGAGGAG GAAAAAGCAG CNNNNN 26

(2) INFORMATION FOR SEQ ID NO: 21:

(i) SEQUENCE CHARACTERISTICS:

(A) LENGTH: 26 nucleotides
(B) TYPE: nucleic acid
(C) STRANDEDNESS: double
(D) TOPOLOGY: linear

(xi) SEQUENCE DESCRIPTION: SEQ ID NO: 21:

NNGGAGGAGG AAAAAGCAGC NNNNNN 26

(2) INFORMATION FOR SEQ ID NO: 22:

(i) SEQUENCE CHARACTERISTICS:

(A) LENGTH: 26 nucleotides
(B) TYPE: nucleic acid
(C) STRANDEDNESS: double
(D) TOPOLOGY: linear

(xi) SEQUENCE DESCRIPTION: SEQ ID NO: 22: .

NGGAGGAGGA AAAAGCAGCN NNNNNN 26

(2) INFORMATION FOR SEQ ID NO: 23:

(i) SEQUENCE CHARACTERISTICS:

(A) LENGTH: 26 nucleotides
(B) TYPE: nucleic acid
(C) STRANDEDNESS: double
(D) TOPOLOGY: linear

(xi) SEQUENCE DESCRIPTION: SEQ ID NO: 23:

GGAGGAGGAA AAAGCAGCNN NNNNNN 26

(2) INFORMATION FOR SEQ ID NO: 24:

(i) SEQUENCE CHARACTERISTICS:

(A) LENGTH: 43 nucleotides
(B) TYPE: nucleic acid
(C) STRANDEDNESS: double
(D) TOPOLOGY: linear

(xi) SEQUENCE DESCRIPTION: SEQ ID NO: 24:

NNNNNNNNAG AGAGAGAGAG GAGAGAGAGA GTAGAGAGGA CCG 43

(2) INFORMATION FOR SEQ ID NO: 25:

(i) SEQUENCE CHARACTERISTICS:

(A) LENGTH: 12 nucleotides
(B) TYPE: nucleic acid
(C) STRANDEDNESS: double
(D) TOPOLOGY: linear

(xi) SEQUENCE DESCRIPTION: SEQ ID NO: 25:

CGGUCCUCUC UA 12

(2) INFORMATION FOR SEQ ID NO: 26:

(i) SEQUENCE CHARACTERISTICS:

(A) LENGTH: 18 nucleotides
(B) TYPE: nucleic acid
(C) STRANDEDNESS: double
(D) TOPOLOGY: linear

(xi) SEQUENCE DESCRIPTION: SEQ ID NO: 26:

AAAAGGAGGA GGCCTTGA 18

(2) INFORMATION FOR SEQ ID NO: 27:

(i) SEQUENCE CHARACTERISTICS:

(A) LENGTH: 18 nucleotides
(B) TYPE: nucleic acid
(C) STRANDEDNESS: double
(D) TOPOLOGY: linear

(xi) SEQUENCE DESCRIPTION: SEQ ID NO: 27:

GGAGGAGGAA AAAGCAGC 18

## Description

### Field of the Invention

The invention relates generally to methods for sequencing polynucleotides, and more particularly, to a method of sorting and sequencing many polynucleotides simultaneously.

### BACKGROUND

The desire to decode the human genome and to understand the genetic basis of disease and a host of other physiological states associated differential gene expression has been a key driving force in the development of improved methods for analyzing and sequencing DNA, Adams et al, Editors, Automated DNA Sequencing and Analysis (Academic Press, New York, 1994). Current genome sequencing projects use Sanger-based sequencing technologies, which enable the sequencing and assembly of a genome of 1.8 million bases with about 24 man-months of effort, e.g. Fleischmann et al, Science, 269: 496-512 (1995). Such a genome is about .005 the size of the human genome, which is estimated to contain about $10^5$ genes, 15% of which--or about 3 megabases--are active in any given tissue. The large numbers of expressed genes make it difficult to track changes in expression patterns by sequence analysis. More commonly, expression patterns are initially analyzed by lower resolution techniques, such as differential display, indexing, subtraction hybridization, or one of the numerous DNA fingerprinting techniques, e.g. Lingo et al, Science. 257: 967-971 (1992); Erlander et al, International patent application PCT/US94/13041; McClelland et al, U.S. patent 5,437,975; Unrau et al, Gene, 145: 163-169 (1994); and the like. Sequence analysis is then frequently carried out on subsets of cDNA clones identified by application of such techniques, e.g. Linskens et al, Nucleic Acids Research, 23: 3244-3251 (1995). Such subsequent analysis is invariably carried out using conventional Sanger sequencing of randomly selected clones from a subset; thus, the scale of the analysis is limited by the Sanger sequencing technique.

Several prior publications by the applicants (PCT Pubn. Nos. WO 9641011, WO 9612039, and WO 9612014) describe sorting and sequencing of populations of polynucleotides. In these disclosures, each polynucleotide is conjugated with an oligonucleotide tag, and the polynucleotide-tag conjugates are sorted by hybridizing the tags to their respective complements on a solid support. Sequencing reactions are carried out on the polynucleotides as they are bound to the solid support.

Two publications by Hitachi Ltd. (EP A 0701001 and EP A 0630792) describe methods of DNA sequence analysis, using oligonucleotide probes, which may be bound to a solid suport, and which have a "common sequence" and a variable "selective sequence" of 1-3 bases. The complement of the common sequence is attached to each of the analyte DNA fragments, and they are then hybridized to the probes via this common sequence. A selective extension reaction is then carried out, and occurs only when the "selective sequence" of the probe is complementary to a portion of the analyte fragment (adjacent to the attached common sequence). Nonextended analytes are then removed by increasing the temperature.

Recently, two techniques have been reported that attempt to provide direct sequence information for analyzing patterns of gene expression, Schena et al, Science, 270: 467-469 (1995)(hybridizing mRNA to 45 expressed sequence tags attached to a glass slide) and Velculescu et al; Science, 270: 484-486 (1995)(excision and concatenation of short tags adjacent to type IIs restriction sites in sequences from a cDNA library, followed by Sanger sequencing of the concatenated tags). However, implementation of these techniques has only involved relative few sequences (45 and 30, respectively) so it is not clear whether they have the capability to track a more meaningful sample of expressed genes, e.g. Kollner et al, Genomics, 23: 185-191 (1994). Without substantially larger sample sizes, the techniques will not be able to track changes in the transcript levels of low-expression genes.

It is clear from the above that there is a crucial need both for higher throughput sequencing techniques that can reduce the time and effort required to analyze genomesized DNAs and that can be applied to the analysis of large samples of sequences from complex mixtures of polynucleotides, such as cDNA libraries. The availability of such techniques would find immediate application in medical and scientific research, drug discovery, diagnosis, forensic analysis, food science, genetic identification, veterinary science, and a host of other fields.

### Summary of the Invention

An object of my invention is to provide a new method and approach for determining the sequence of poiynucleotides.

Another object of my invention is to provide a method for rapidly analyzing patterns of gene expression in normal and diseased tissues and cells.

A further object of my invention is to provide a method for simultaneously analyzing and/or sequencing a population of many thousands of different polynucleotides, such as a sample of polynucleotides from a cDNA library or a sample of fragments from a segment of genomic DNA.

Still another object of my invention is to provide a method for identifying populations of polynucleotides.

Another object of my invention is to provide a method for sequencing segments of DNA in a size range corresponding to typical cosmid or YAC inserts.

My invention achieves these and other objectives by providing'each polynucleotide of a population with an oligonucleotide tag for transferring sequence information to a tag complement on a spatially addressable array of such complements. That is, a unique tag is attached to each polynucleotide of a population which can be cleaved or copied and used to shuttle sequence information to its complement at a fixed position on an array of such complements. After a tag specifically hybridizes with its complement, a signal is generated that is indicative of the transferred sequence information. Preferably, the invention is carried but by repeated cycles of amplifying the polynucleotides, identifying one or more nucleotides at an end of each polynucleotide by use of the oligonucleotide tags, and shortening the polynucleotides by removing one or more nucleotides.

At least two major advantages are gained by using tags to shuttle information to discrete spatial locations rather than sorting an entire population of polynucleotides to such locations: First, tags are much smaller molecular entities so that the kinetics of diffusion and hybridization are much more favorable. Second, tag loading at the spatially discrete locations only need be sufficient for detection, while polynucleotide loading would need to be sufficient for both biochemical processing and detection; thus, far less tag needs to be loaded on the spatially discrete sites.

An important aspect of the invention is the attachment of an oligonucleotide tag to each polynucleotide of a population such that substantially all different polynucleotides have different tags. As explained more fully below, this is achieved by taking a sample of a full ensemble of tag-polynucleotide conjugates wherein each tag has an equal probability of being attached to any polynucleotide. The sampling step ensures that the tag-polynucleotide conjugate population will fulfill the above-stated condition that the tag of any polynucleotide of such population be substantially unique.

Complements of the oligonucleotide tags are preferably synthesized on the surface of a solid phase support, such as a microscopic bead or a specific location on an array of synthesis locations on a single support, such that populations of identical sequences are produced in specific regions. That is, the surface of each support, in the case of a bead, or of each region, in the case of an array, is derivatized by only one type of tag complement which has a particular sequence. The population of such beads or regions contains a repertoire of complements with distinct sequences. As used herein in reference to oligonucleotide tags and tag complements, the term "repertoire" means the set of minimally cross-hybridizing set of oligonucleotides that make up the tags in a particular embodiment or the corresponding set of tag complements.

My invention provides a readily automated system for obtaining sequence information from large numbers of polynucleotides at the same time. My invention is particularly useful in operations requiring the generation of massive amounts of sequence information, such as large-scale sequencing of genomic DNA fragments, mRNA and/or cDNA fingerprinting, and highly resolved measurements of gene expression patterns.

## Brief Description of the Drawings

Figure 1 is a flow chart illustrating a general algorithm for generating minimally cross-hybridizing sets.
Figure 2a illustrates the major steps of the preferred embodiment of the method of the invention.
Figure 2b illustrates the use of rolling primers in the method of the invention.
Figure 2c illustrates the manner in which rolling primers are selected in successive cycles of amplification, tag transfer, and template mutation.
Figure 2d illustrates the use of S and T primers in one embodiment of the invention.
Figure 3 diagrammatically illustrates an apparatus for detecting labeled tags on a spatially addressable array of tag complements.

## Definitions

"Complement" or "tag complement" as used herein in reference to oligonucleotide tags refers to an oligonucleotide to which a oligonucleotide tag specifically hybridizes to form a perfectly matched duplex or triplex. In embodiments where specific hybridization results in a triplex, the oligonucleotide tag may be selected to be either double stranded or single stranded. Thus, where triplexes are formed, the term "complement" is meant to encompass either a double stranded complement of a single stranded oligonucleotide tag or a single stranded complement of a double stranded oligonucleotide tag.

The term "oligonucleotide" as used herein includes linear oligomers of natural or modified monomers or linkages, including deoxyribonucleosides, ribonucleosides, -anomeric forms thereof, peptide nucleic acids (PNAs), and the like, capable of specifically binding to a polynucleotide by way of a regular pattern of monomer-to-monomer interactions, such as Watson-Crick type of base pairing, base stacking, Hoogsteen or reverse Hoogsteen types of base pairing, or the like. Usually monomers are linked by phosphodiester bonds or analogs thereof to form oligonucleotides ranging in size from a few monomeric units, e.g. 3-4, to several tens of monomeric units. Whenever an oligonucleotide is represented by a sequence of letters, such as "ATGCCTG," it will be understood that the nucleotides are in 5'→3' order from left to right and that "A" denotes deoxyadenosine, "C" denotes deoxycytidine, "G" denotes deoxyguanosine, and "T" denotes thymidine, unless otherwise noted. Analogs of phosphodiester linkages include phosphorothioate, phosphorodithioate, phosphoranilidate, phosphoramidate, and the like. It is clear to those skilled in the art when oligonucleotides having natural or non-natural nucleotides may be employed, e.g. where processing by enzymes is called for, usually oligonucleotides consisting of natural nucleotides are required.

"Perfectly matched" in reference to a duplex means that the poly- or oligonucleotide strands making up the duplex form a double stranded structure with one other such that every nucleotide in each strand undergoes Watson-Crick basepairing with a nucleotide in the other strand. The term also comprehends the pairing of nucleoside analogs, such as deoxyinosine, nucleosides with 2-aminopurine bases, and the like, that may be employed. In reference to a triplex, the term means that the triplex consists of a perfectly matched duplex and a third strand in which every nucleotide undergoes Hoogsteen or reverse Hoogsteen association with a basepair of the perfectly matched duplex. Conversely, a "mismatch" in a duplex between a tag and an oligonucleotide means that a pair or triplet of nucleotides in the duplex or triplex fails to undergo Watson-Crick and/or Hoogsteen and/or reverse Hoogsteen bonding.

As used herein, "nucleoside" includes the natural nucleosides, including 2'-deoxy and 2'-hydroxyl forms, e.g. as described in Kornberg and Baker, DNA Replication, 2nd Ed. (Freeman, San Francisco, 1992). "Analogs" in reference to nucleosides includes synthetic nucleosides having modified base moieties and/or modified sugar moieties, e.g. described by Scheit, Nucleotide Analogs (John Wiley, New York, 1980); Uhlman and Peyman, Chemical Reviews, 90: 543-584 (1990), or the like, with the only proviso that they are capable of specific hybridization. Such analogs include synthetic nucleosides designed to enhance binding properties, reduce complexity of probes, increase specificity, and the like.

As used herein, "amplicon" means the product of an amplification reaction. That is, it is a population of identical polynucleotides, usually double stranded, that are replicated from a few starting sequences. Preferably, amplicons are produced in a polymerase chain reaction (PCR).

As used herein, "complexity-reducing nucleotide" refers to a natural or non-natural nucleotide (i) that, when paired with either of more than one natural nucleotides, can form a duplex of substantially equivalent stability to that of the same duplex containing cognate natural nucleotide--i.e. the natural nucleotide it replaces, and (ii) that can be processed by enzymes substantially the same as its cognate natural nucleotide. Preferably, complexity-reducing nucleotides do not display degeneracy or ambiguity when processed by DNA polymerases. That is, when a complexity-reducing nucleotide is in a template that is being copied by a polymerase, the polymerase incorporates a unique nucleotide at the site of a complexity-reducing nucleotide. Likewise, when a complexity-reducing nucleotide triphosphate is a substrate for a DNA polymerase, it is incorporated only at the site of a single kind of nucleotide, i.e. one or another of its complements, but not both. Candidate complexity-reducing nucleotides are readily tested in straight forward hybridization assays, e.g. with melting temperature comparisons, and in incorporation assays in which test polymerizations are checked by conventional sequencing or by incorporation of radio-labeled complexity-reducing nucleotides, e.g. Bessman et al, Proc. Natl. Acad. Sci., 44: 633 (1958). Preferably, "substantially equivalent stability," as used herein means that the melting temperature of a test 13-mer duplex, as described in Kawase et al, Nucleic Acids Research, 14: 7727-7736 (1986), is within twenty percent of that of the same duplex containing a natural cognate nucleotide.

## Detailed Description of the Invention

The invention provides a method of sequencing large numbers of polynucleotides in parallel by using oligonucleotide tags to shuttle sequence information obtained in "bulk" or solution phase biochemical processes to discrete spatially addressable sites on a solid phase. Signals generated at the spatially addressable sites convey the sequence information carried by the oligonucleotide tag. As explained more fully below, sequencing is preferably carried out by repeated cycles of amplifying the polynucleotides, identifying nucleotides, and shortening the polynucleotides. In the shortening cycles, a predetermined number of nucleotides (usually the nucleotides identified in the previous cycle) are cleaved from the polynucleotides and the shortened polynucleotides are employed in the next cycle of amplification, identification, and shortening. Since the oligonucleotide tags specifically hybridize to the same location on a spatially addressable array of tag complements, the sequence of a particular polynucleotide can be read by observing the signals generated from that location through successive cycles of the method.

More particularly, the invention is carried out by the following steps: (a) attaching an oligonucleotide tag from a repertoire of tags to each polynucleotide of a population to form tag-polynucleotide conjugates such that substantially all different polynucleotides have different oligonucleotide tags attached; (b) providing a label for each oligonucleotide tag for identifying one or more terminal nucleotides of its associated polynucleotide; (c) transferring the labeled oligonucleotide tags or copies thereof onto a spatially addressable array of tag complements for sorting and specific hybridization of the oligonucleotide tags or copies thereof and detection of the labels. Preferably, the method further includes the steps of (d) shortening the polynucleotides so that further sequence information can be obtained and (e) repeating steps (b)-(d) until sufficient sequence information is accumulated for identification of the polynucleotides. Preferably, the step of transferring the oligonucleotide tags to the array of tag complements includes separating the oligonucleotide tags from the amplification reaction mixture, cleaving the oligonucleotide tags from their tag-polynucleotide conjugates, and applying the oligonucleotide tags to the array of tag complements.

Preferably, the step of providing a label for the oligonucleotide tags is accomplished by selectively amplifying polynucleotides in a polymerase chain reaction. As illustrated in Figure 2a, in the preferred embodiment, a population of tagged polynucleotides (200) having flanking primer binding sites (12 & 22) is aliquotted into four reaction vessels (28)-(34) containing common, or "T" primers (210), and "S," or second, primers which have defined 3' terminal nucleotides of A, C, G, and T, respectively (202)-(208). Preferably, a T primer carries a fluorescent label indicative of the defined 3' terminal nucleotide of the S primer in its reaction vessel. After amplification (207), an aliquot of the amplicons is taken (209) from each vessel, pooled, and the labeled oligonucleotide tags are prepared for transferring to a spatially addressable array (48). Separately, aliquots are also taken from each vessel, pooled, and shortened (213) by mutating (if necessary) or cleaving the polynucleotide to remove the nucleotide(s) just identified. The shortened polynucleotides are then re-aliquotted (214) to four reaction vessels for the next cycle of selective amplification, transfer of labeled tags, and shortening. In successive cycles, tag complement locations (216) receiving labeled oligonucleotide tags will successively generate signals indicative of the defined 3' terminal nucleotides of the S primers.

In one embodiment, the identity of the one or more terminal nucleotides is determined by selectively amplifying correct sequence primers in a polymerase chain reaction (PCR) employing primers whose 3' terminal sequences are complementary to every possible sequence of the one or more terminal nucleotides whose identity is sought. Thus, when the identity of a single terminal nucleotide is sought, four separate polymerase chain reactions may be carried out with one primer identical in each of the four reactions, but with each of the other four primers having a 3' terminal nucleotide that is either A, C, G, or T. As used herein, this terminal nucleotide is referred to as a "defined 3' terminal nucleotide." The defined 3' terminal nucleotide is positioned so that it must be complementary to the terminal nucleotide of the polynucleotide for amplification to occur. Thus, the identity of the primer in a successful amplification gives the identity of the terminal nucleotide of the target sequence. This information is then extracted in parallel from the population of polynucleotides by detaching or copying the amplified tags and sorting them onto their tag complements on a spatially addressable array. After amplification and identification, the polynucleotides are shortened by removing one or more terminal nucleotides, for example, by cleavage with a type IIs restriction endonuclease. By repeating this process for successive nucleotides the sequences of a population of polynucleotides are determined in parallel.

In another embodiment, the step of shortening is accomplished by advancing a primer along the polynucleotide templates by template mutation. An important feature of this embodiment is providing a set of primers, referred to herein as "rolling primers" that contain complexity-reducing nucleotides for reducing the number of primers required for annealing to every possible primer binding site on templates formed from the polynucleotides. Another important feature of this embodiment is the systematic replacement of at least one of the four nucleotides in the polynucleotide with its cognate complexity-reducing nucleotide or complement thereof. Sequencing is initiated by annealing rolling primers differing only in their terminal nucleotides to a primer binding site of the polynucleotide templates so that only the rolling primer whose terminal nucleotide forms a perfect complement with the template leads to the formation of an extension product. After amplifying the double stranded extension product to form an amplicon, the terminal nucleotide, and hence its complement in the template, is identified by the identity of the amplicon. For example, in a form of this embodiment, a terminal nucleotide may be identified by the presence or absence of amplicon in four vessels that are used for separate extension and amplification reactions. The primer binding site of the template of the successfully amplified polynucleotide is then mutated by, for example, oligonucleotide-directed mutagenesis so that a subsequent rolling primer may be selected from the set that forms a perfectly matched duplex with the mutated template at a site which is shifted towards the direction of extension by one nucleotide relative to the binding site of the previous rolling primer. The steps of selective extension, amplification and identification are then repeated. In this manner, the primers "roll" along the polynucleotide during the sequencing process, moving a base at a time along the template with each cycle. Preferably, in the rolling primer embodiment the tag-polynucleotide conjugate is shortened by a single nucleotide in each cycle of steps.

The preferred embodiments and common elements of the invention are described in more detail below.

<u>Rolling Primers</u>

Preferably, rolling primers are from 15 to 30 nucleotide in length and have the following form:

$$X_1X_2 \ldots \underline{X_k}YY \ldots YN$$

where the $X_i$'s are nucleotides, preferably arranged in repetitive subunits; Y's are complexity-reducing nucleotides or their complements; and N is a terminal nucleotide of either A, C, G, or T, or a complexity -reducing nucleotide, such as deoxyinosine. The segments of $X_i$ nucleotides, referred to herein as the "template positioning segments," are preferably arranged in repetitive subunits so that the primer is properly registered on the primer binding site with the terminal nucleotide juxtaposed with the first nucleotide of polynucleotide. Preferably, the repeat subunit is long enough so that if the primer is out of register by one or more repeat subunits, it will be too unstable to remain annealed to the template. Preferably, the repeat subunit is from 4 to 8 nucleotides in length. As will become more apparent below, arranging the template positioning segment as a series of identical subunits reduces the overall number of primers required in a set of rolling primers. Preferably, the template positioning segments are selected from a group of no more than two nucleotides, at least one of which is a complement of a complexity-reducing nucleotide being employed. In preferred embodiments, the underlined $X_k$ indicates the position at which the template is mutated by way of oligonucleotide-directed mutagenesis, e.g. a technique fully described in Current Protocols in Molecular Biology (John Wiley & Sons, New York, 1995).

The segment YY ... YN is referred to herein as the "extension region" of the primer, as the primer is extended from this end along the template. Preferably, extension is carried out by a polymerase so that YY ... YN is in a 5'→3' orientation. However, the orientation could be 3'→5' with other methods of extension, e.g. by ligating oligonucleotide blocks as described U.S. patent 5,114,839. An important feature of the invention is that extension only take place when the terminal nucleotide, N, forms a Watson-Crick base pair with the adjacent nucleotide in the template. The extension region comprises the minimal number of nucleotides greater than two that can form a stable duplex with the template, even if there is a mismatch at the $X_k$ position. That is, in the preferred embodiments, the duplex between the extension region and the template must be stable enough to carry out the oligonucleotide-directed mutagenesis. Preferably, the extension region comprises from 3 to 6 nucleotides, and most preferably, it comprises 4 nucleotides. Preferably, Y is selected from the group consisting of deoxyadenosine (A) and deoxyinosine (I).

The number of rolling primers required for a particular embodiment depends on several factors, including the type of complexity-reducing nucleotides employed, the length of the primer, the length of the extension region, and the repeat subunit length of the template positioning segment. For example, the following set of primers (SEQ ID NO: 1-6) has a template positioning segment 18 nucleotides in length made up of subunits of G's and A's 6 nucleotides in length.

| Subgroup | Rolling Primer Sequence |
|---|---|
| (1) | GGAAGAGGAAGAGGAAGAYYYN |
| (2) | GAAGAGGAAGAGGAAGAGYYYN |
| (3) | AAGAGGAAGAGGAAGAGGYYYN |
| (4) | AGAGGAAGAGGAAGAGGAYYYN |
| (5) | GAGGAAGAGGAAGAGGAAYYYN |
| (6) | AGGAAGAGGAAGAGGAAGYYYN |

If Y is A or I and N is A, C, I, or T, then the above set of rolling primers includes 192 (=6 x $2^3$ x 4) primers. In particular, each "YYY" represents all of the following sequences: AAA, AAI, AII, AIA, IAI, IAA, IIA, and III. As can be seen from the above example, a template positioning segment is available for shifting the primer one nucleotide in the direction of extension after any cycle. That is, if a primer from subgroup (5) were employed in a cycle, the next primer employed would be selected from subgroup (6), if a primer from subgroup (6) were employed in a cycle, the next primer employed would be selected from subgroup (1), and so on. When PCR is used to copy and amplify the template, the template is, in effect, shortened by one nucleotide in each cycle.

Alternatively, the binding strength of the extension region can be improved by substituting G for I and diaminopurine (D) for A in all positions, except those immediately adjacent to the terminal nucleotide. That is, an alternative set of "YYY" sequences include DDA, DDI, DGI, DGA, GDI, GDA, GGI, and GGA.

Sequencing with Rolling Primers

Generally. this aspect of my invention is carried out with the following steps: (a) providing a set of primers, i. e. the rolling primers, each primer of the set having an extension region comprising one or more complexity-reducing nucleotides and a terminal nucleotide; (b) forming a template comprising a primer binding site and the polynucleotide whose sequence is to be determined, the primer binding site being complementary to the extension region of at least one primer of the set; (c) annealing a primer from the set to the primer binding site, the extension region of the primer forming a perfectly matched duplex with the template and extending the primer to form a double stranded DNA; (d) amplifying the double stranded DNA to form an amplicon; (e) identifying the terminal nucleotide of the extension region of the primer by the identity of the amplicon; (f) mutating the primer binding site of the template so that the primer binding site is shifted one or more nucleotides in the direction of extension, thereby effectively shortening the polynucleotide by one or more nucleotides; and (g) repeating steps (c) through (f) until the nucleotide sequence of the polynucleotide is determined.

Prior to sequencing, a polynucleotide is treated so that one or more kinds of nucleotide are substituted with their cognate complexity-reducing nucleotides. In a preferred embodiment, this is conveniently accomplished by replicating the polynucleotide in a PCR wherein dGTP is replaced with dITP. A template for sequencing is then prepared by joining the polynucleotide to a primer binding site. Typically, this is accomplished by inserting the polynucleotide into a vector which carries the primer binding site. Preferably, the primer binding site is in the 3' direction relative to the polynucleotide so that primer extensions can be carried out with a DNA polymerase. Such insertion is conveniently carried out using a blunt-end-cutting restriction endonuclease, such as Stu I or Eel 136 II, if the rolling primers described above are employed. These enzymes leave a three-base sequence adjacent to the beginning of-the polynucleotide that is complementary to the primers described above. Preferably, a primer, referred herein as the "T" primer, is located at the other end of the polynucleotide so that it can be amplified by PCR. For example, sequencing can be initiated on such a template in four separate reactions as shown below, assuming the use of the primers (SEQ ID NO: 1-6) described above.

```
Reaction 1          GGAAGAGGAAGAGGAAGAAIIA->
                ... CCTTCTCCTTCTCCTTCTTCCNNNN ... NNNBBBB ... BB ...

Reaction 2          GGAAGAGGAAGAGGAAGAAIIC->
                ... CCTTCTCCTTCTCCTTCTTCCNNNN ... NNNBBBB ... BB ...

Reaction 3          GGAAGAGGAAGAGGAAGAAIII->
                ... CCTTCTCCTTCTCCTTCTTCCNNNN ... NNNBBBB ... BB ...

Reaction 4          GGAAGAGGAAGAGGAAGAAIIT->
                ... CCTTCTCCTTCTCCTTCTTCCNNNN ... NNNBBBB ... BB ...
```

where "NNNN ... NNN" represents the polynucleotide and "BBBB ... BB" represents the complement of a T primer binding site for amplifying the sequences by PCR. The underlined sequences indicate the extension regions of the rolling primers. The template positioning segment of the primers was arbitrarily chosen to correspond to a primer from subgroup (1) described above. If it is assumed--to illustrate the method-- that the sequence of the polynucleotide adjacent to the rolling primer binding site is "TAIC," then only Reaction 1 will result in the formation of an amplicon, and the first nucleotide of the polynucleotide is identified as T. Preferably, prior to amplification, the primer is extended with a high fidelity DNA polymerase, such as Sequenase, in the presence of dATP, dCTP, dITP, and dTTP in the preferred embodiments. It should be understood that selective extension may also be carried out in a single vessel, for example, if labeled primers are employed and the extension products are separated from the primers that fail to extend. The important feature is that only primers whose terminal nucleotide forms a correct Watson-Crick basepair with the template are extended. Preferably, after extension, any single stranded DNA in the reaction mixture is digested with a single stranded nuclease, such as Mung bean nuclease. After such extension and digestion, the remaining double stranded DNA is then amplified, again in the presence of dATP, dCTP, dITP, and dTTP in the preferred embodiments, to produce an amplicon. Preferably. this amplification is accomplished by 5-10 cycles of PCR so that there is little or no likelihood of anomalous amplification products being produced.

Samples of the amplicon from Reaction 1 are removed and aliquotted into four new vessels containing following primers from subgroup (2)(SEQ ID NO: 2):

```
Reaction 5           GAAGAGGAAGAGGAAGAGIIAA->
                 ... CCTTCTCCTTCTCCTTCTTCCTNNN ... NNNBBBB ... BB

Reaction 6           GAAGAGGAAGAGGAAGAGIIAC->
                 ... CCTTCTCCTTCTCCTTCTTCCTNNN ... NNNBBBB ... BB

Reaction 7           GAAGAGGAAGAGGAAGAGIIAI->
                 ... CCTTCTCCTTCTCCTTCTTCCTNNN ... NNNBBBB ... BB

Reaction 8           GAAGAGGAAGAGGAAGAGIIAT->
                 ... CCTTCTCCTTCTCCTTCTTCCTNNN ... NNNBBBB ... BB
```

Since the first nucleotide of the polynucleotide was determined in the previous cycle, one selects primers from subgroup (2) whose extension regions have the form "IIAN," as shown. This creates a mismatch at the underlined T in the lower strands, which is mutated to C in any amplicon produced by oligonucleotide-directed mutagenesis. That is, the primer is the oligonucleotide directing the mutation of the site in the amplicon. Thus, the "T" is converted into a "C" in the amplicons. Since the second nucleotide of the target is A, both Reactions 7 and 8 lead to the production of amplicons. Either amplicon may be sampled for the next cycle since only a single polynucleotide is presently being considered. As explained more fully below, an additional "pooling" step must be carried out when multiple polynucleotides are simultaneously sequenced.

As before, samples of one of the two amplicons are distributed into four new vessels containing primers from subgroup (3) (SEQ ID NO: 3) with an extension region having the form "IAIN".

```
Reaction 9           AAGAGGAAGAGGAAGAGGIAIA->
                 ... CCTTCTCCTTCTCCTTCTCCCTANN ... NNNBBBB ... BB

Reaction 10          AAGAGGAAGAGGAAGAGGIAIC->
                 ... CCTTCTCCTTCTCCTTCTCCCTANN ... NNNBBBB ... BB

Reaction 11          AAGAGGAAGAGGAAGAGGIAII->
                 ... CCTTCTCCTTCTCCTTCTCCCTANN ... NNNBBBB ... BB

Reaction 12          AAGAGGAAGAGGAAGAGGIAIT->
                 ... CCTTCTCCTTCTCCTTCTCCCTANN ... NNNBBBB ... BB
```

Both Reactions 9 and 10 will produce amplicons; thus, the third base is identified as an "I." For the next cycle, this then leads to the selection of primers from subgroup (4) having an extension region with the form "AIAN," and the process is continued.

Sequencing Tagged Polynucleotides Using Rolling Primers

A preferred embodiment for simultaneously sequencing a population of tagged polynucleotides is diagrammed in Fig. 2b. Preferably, the population of tagged polynucleotides is amplified from a vector as described above in the presence of dATP, dCTP, dITP, and dTTP to give a population of double stranded DNAs (10) containing T primer binding site (12), cleavage site (14)--which as shown below is optional, tag (16), cleavage site (18), polynucleotides (20), and rolling primer binding site (22). The population of double stranded DNAs (10) is methylated so that when tags are later excised only the double stranded DNAs that have been selectively amplified--and therefore lack methylated bases--will be cleaved to yield tags for detection.

In the initial population, rolling primer binding site (22) contains a known complement to the extension region (24), for example, AGG as shown in the example below. Samples of the initial population are preferably transferred (26) to four separate vessels (28-34) where they are combined with the rolling primers of subgroup (1), described above, having extension regions -AIIA, -AIIC, -AIIG, and -AIIT. (The four rolling primer could be placed in a single vessel and allowed to compete against one another for extension; however, errors are less likely if the primers are used separately). The rolling primers of subgroups (1)-(6) are used here to exemplify the invention. Clearly, many alternative forms of the rolling primers could be used. In subsequent cycles, as described more fully below, the transferring step (26) becomes more complex because more than four vessels, i.e. up to 32 (=4 x 8) in the embodiment exemplified here, are required for the extension reactions. After the double stranded DNAs (10) are combined with the appropriate rolling primers the following steps (36) are taken: the double stranded DNAs are denatured, e.g. by heating; the temperature is lowered to permit the rolling primers to anneal to the rolling primer binding sites; the primers are extended with a high fidelity DNA polymerase, such as Sequenase, in the presence of dATP, dCTP, dITP, and dTTP; preferably, any remaining single stranded DNA is digested, e.g. with a single stranded nuclease, such as Mung bean nuclease, to reduce the likelihood of interference from the left over single stranded DNA in the subsequent amplification; T primer is added; and the double stranded extension products are amplified, preferably with 5-10 cycles of PCR, to generate amplicon A (38), amplicon C (40), amplicon G (42), and amplicon T (44), respectively.

After a sample is taken from each amplicon, tags are excised by way of cleavage sites (14) and (18) and labeled (46), as described more fully below. The labeled tags are then either applied separately to their tag complements on solid phase support (48) or pooled and applied to the support, depending on the labeling system employed, the complexity of the tag mixture, and like factors. Samples of the amplicons are also taken for further processing (50-56) in accordance with the method of the invention. Depending on the identity of the most recently determined nucleotide and the identity of the current extension region, a sample either may be separately aliquotted into vessels with rolling primers for the next cycle, or a sample may be combined with one or more other samples and aliquotted into vessels with rolling primers for the next cycle. Unlike the single polynucleotide case, when a population of polynucleotides is sequenced every vessel will almost always contain an amplicon at the conclusion of the amplification reaction. Thus, after extension, digestion, and amplification, the amplicons in the vessels 28, 30, 32, and 34 correspond to polynucleotides having a T, G (or I), C, and A at their initial positions (or more generally, at the nucleotide position adjacent to the rolling primer binding site), respectively. With this information, and a knowledge of the sequence of the extension region of the current amplicon, the rolling primers of the next cycle can be selected. As in the single polynucleotide case, in each successive cycle a rolling primer is selected that shifts, or advances, the rolling primer binding site one or more nucleotides along the template in the direction of rolling primer extension. Preferably, a single nucleotide shift takes place in each cycle. As described above, the rolling primers selected for the extension step also serve to generate a mutation in the template upon amplification. The mutation changes the interior-most nucleotide of the extension region to one that is complementary to the template positioning segment of the rolling primer of the current cycle. In the tables below, the pattern of primer selection and amplicon pooling in cycles 2 through 4 of a sequencing operation is illustrated for the above embodiment. In the first cycle, the original template is distributed to four vessels for denaturation and extension.

**Selection of Rolling Primers for 2nd Cycle**

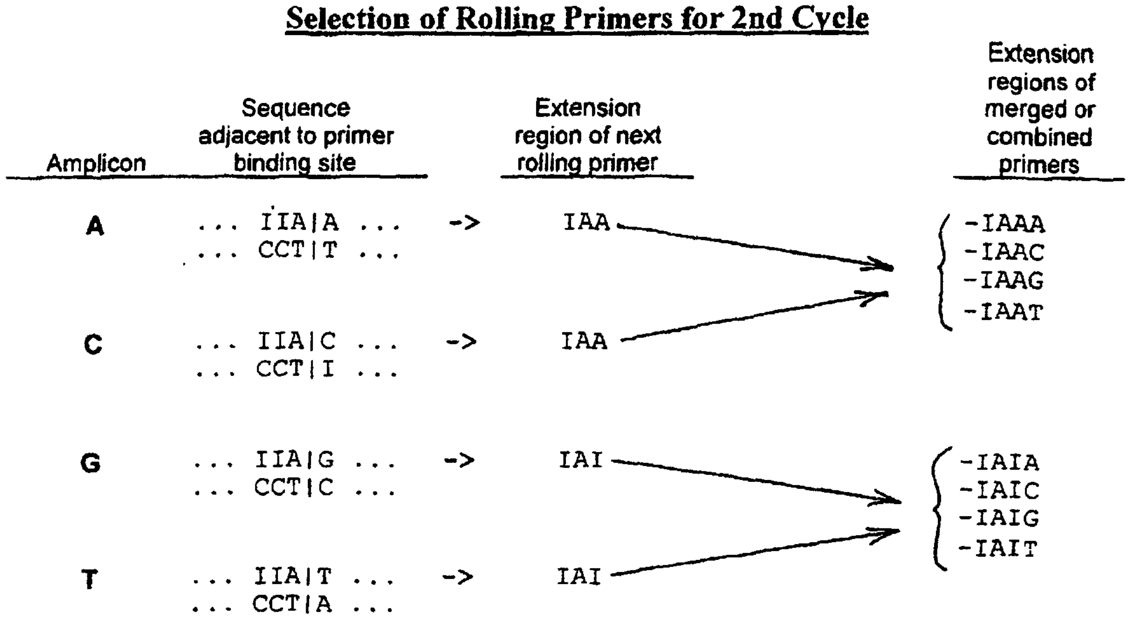


The nucleotide to the right of the line between nucleotides in the second column is the terminal nucleotide of the rolling primer used to produce the amplicon. Generally, the algorithm for determining the rolling primers of the next cycle is as follows: (i) drop the nucleotide distal to the terminal nucleotide in the extension region of the current rolling primer (the leftmost "I" of the "IIA" sequences in the second column), (ii) determine which nucleotide, I or A, is complementary to the nucleotide paired with the terminal nucleotide (i.e. for the above example: "A" for amplicon A, "A" for amplicon C--since A will pair with I as well as T, "I" for amplicon G--since I will pair with C, and "I" for amplicon T--since I will also pair with A), (iii) insert the determined nucleotide, I or A, to the left of the terminal nucleotide. For this embodiment. the general pattern of transitions between extension region sequences is illustrated in Figure 2b. Longer extension regions lead to more complex patterns, but the basic algorithm defining permissible transitions remains the same.

## Selection of Rolling Primers for 3rd Cycle

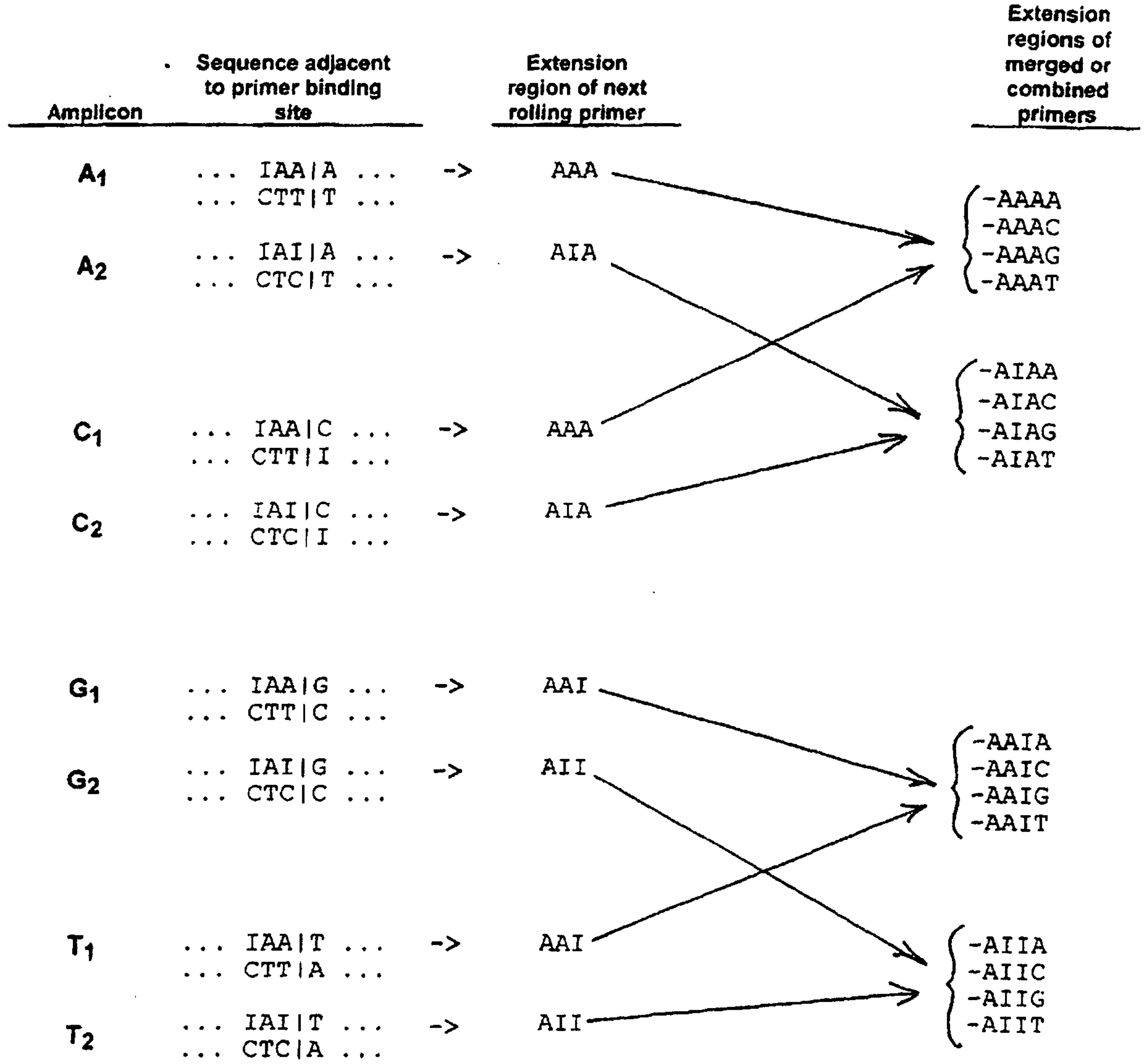

## Selection of Rolling Primers for 4th Cycle

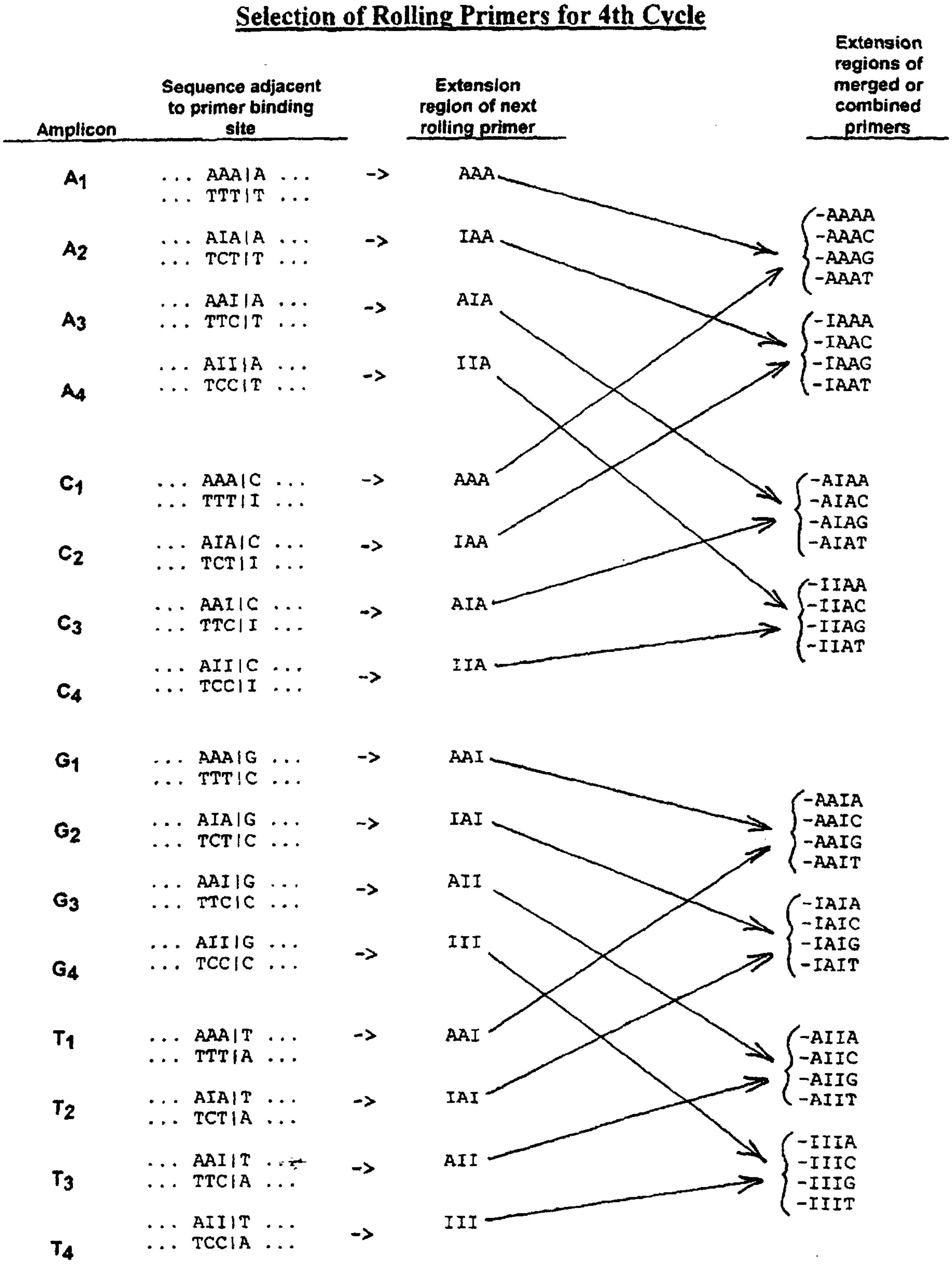


Typically, by the eighth cycle thirty-two reactions are required, and continue to be required, in each cycle until sequencing is halted.

Clearly, additional steps to those outlined above may be implemented, for example, to separate the initial extension product from extraneous single stranded DNA and/or the single stranded nuclease, if one is employed. Manipulation of polynucleotides and other reagents, temperature control for PCRs, and the like, may be carried out on commercially available laboratory robots, e.g. Biomek 1000 (Beckman Instruments, Fullerton, CA).

Rolling primers and T primers may be constructed to have a double stranded segment capable of binding to an anchored single stranded oligonucleotide via triplex formation for separation, e.g. as taught by Ji et al, Anal. Chem. 65: 1323-1328 (1993); Cantor et al, U.S. patent 5,482,836; or the like. Thus, for example, magnetic beads carrying such a single stranded oligonucleotide can be used to capture the amplicons and transfer them to a separate vessel containing a nuclease to cleave the tag, e.g. at cleavage site 18, of those double stranded DNAs that have been selectively amplified (other DNAs remain unamplified and therefore hemi-methylated so no cleavage occurs). Preferably, the T primer contains a 5' biotin which permits the released tag to be captured and conveniently labeled. After capture, e.g. via avidinated magnetic beads, the 3' strands of the double stranded segment are stripped back to the tag by the use of T4 DNA polymerase, or like enzyme, in the presence of a deoxynucleoside triphosphate (dNTP) corresponding to the nucleotide flanking the tag. Thus, provided that the flanking nucleotides are not present elsewhere along the strand to the 3' ends, the 3'$\rightarrow$5' exonuclease activity of the polymerase will strip back the 3' strand to the flanking nucleotides, at which point an exchange reaction will be initiated that prevents further stripping past the flanking nucleotides. The 3' ends of the tag can then be labeled in an extension reaction with labeled dNTPs. After labeling the non-biotinylated strand can be removed by denaturation and applied to the spatially addressable array for detection.

After the labeled tags are hybridized to their tag complements and detected, the tags are removed by washing so that labeled tags from the next set of amplicons can be applied.

Sequencing Tagged Polynucleotides with S and T Primers

In a preferred embodiment of the invention, the identification of the terminal nucleotides of polynucleotide inserts is accomplished by selectively amplifying sequences that form perfectly matched duplexes with the 3' end of the S primers. As illustrated in Fig. 2d. in each identification cycle multiple sets of S primers are used with the same T primer to produce PCR amplicons. Segments of cloning vector 410 containing T primer binding site 412, cleavage site 414, tag 416, cleavage site 418, polynucleotide 420, and S primer binding site 422 are amplified in separate PCRs for each of the different S primers, which in Fig. 2d number 4k. The S primers of sets 1 through k form duplexes with the S primer binding site and 1 to k terminal nucleotides of the polynucleotide, respectively. In Fig. 2d, "N" represents a mixture of the four natural nucleotides, A, C, G, and T. Thus, the S primers of set 2 are each mixtures of 4 primers: one having a 3' terminal A and a penultimate nucleotide of A, C, G, or T; one having a 3' terminal C and a penultimate nucleotide of A, C, G, or T; one having a 3' terminal G and a penultimate nucleotide of A, C, G, or T; and one having a 3' terminal T and a penultimate nucleotide of A, C, G, or T. In sets 3 through k, "B" represents a complexity-reducing analog, or a mixture of such analogs and natural nucleotides. For example, B could consist of C and deoxyinosine. Such analogs are well known and are describe in Kong Thoo Lin et al, Nucleic Acids Research, 20: 5149-5152; U.S. patent 5,002,867; Loakes et al, Nucleic Acids Research, 22: 4039-4043 (1994); Nichols et al, Nature, 369: 492-493 (1994); and like references. The penultimate N's in S primers 2 through k could also be complexity reducing analogs, provided that the presence of such an analog had no effect on the base pairing or extension of the terminal nucleotide. Indeed, it is not crucial that an S primer form a perfectly matched duplex with its binding site and polynucleotide over the primer's entire length. It is only required that the terminal nucleotide base pair correctly. Preferably, the S and T primers have approximately equal melting and annealing temperatures, and are in the range of 12 to 30 nucleotides in length.

After carrying out the PCRs, k sets of 4 amplicons are produced. The 4k amplicons carry the following information: In set 1, amplicons made with S primers having a terminal A indicate polynucleotides whose first nucleotide is T; amplicons made with S primers having a terminal C indicate polynucleotides whose first nucleotide is G; and so on. In set 2, amplicons made with S primers having a terminal AN indicate polynucleotides whose second nucleotide is T; amplicons made with S primers having a terminal CN indicate polynucleotides whose second nucleotide is G; and so on. Likewise, for amplicons of sets 3 through k, the identities of the nucleotides of the 3rd through kth positions are indicated, respectively. To extract this information, the tags in the amplicons must be excised, labeled, rendered single stranded, and hybridized to their tag complements on a spatially addressable array. Tags 416 are excised by cleaving with restriction endonucleases directed to sites 414 and 418. Preferably, the T primer is constructed to have a means for isolating the amplicons. such as a biotin moiety or a double stranded segment which can form a triplex structure with an anchored single stranded oligonucleotide. Preferably, after separation, the tags of each of the four amplicons of each set are separately labeled, e.g. with a spectrally resolvable fluorescent dye. Thus, all the tags in amplicon made from S primers terminating in A have the same label which can be distinguished from the labels for C, G, and T. The same holds for tags in amplicons made from S primers terminating in C, G, and T, regardless of the set number. Thus, when the tags of the nth set are simultaneously applied to the spatially addressable sites, the identity of the nucleotides at the nth position of all the polynucleotides are determined.

Tags can be labeled in a variety of ways, including the direct or indirect attachment of radioactive moieties, fluorescent moieties, colorimetric moieties, chemiluminescent markers, and the like. Many comprehensive reviews of methodologies for labeling DNA and constructing DNA probes provide guidance applicable to labelling tags of the present invention. Such reviews include Kricka, editor, Nonisotopic DNA Probe Techniques (Academic Press, San Diego, 1992); Haugland, Handbook of Fluorescent Probes and Research Chemicals (Molecular Probes, Inc., Eugene, 1992); Keller and Manak, DNA Probes, 2nd Edition (Stockton Press, New York, 1993); and Eckstein, editor, Oligonucleotides and Analogues: A Practical Approach (IRL Press, Oxford, 1991); Kessler, editor, Nonradioactive Labeling and Detection of Biomolecules (Springer-Verlag, Berlin, 1992); and the like.

Preferably, the tags are labeled with one or more fluorescent dyes, e.g. as disclosed by Menchen et al, U.S. patent 5,188,934; and Begot et al International application PCT/US90/05565.

Preferably, the S primers are constructed to have a double stranded segment capable of binding to an anchored single stranded oligonucleotide for separation, e.g. as taught by Ji et al, Anal. Chem. 65: 1323-1328 (1993). Thus, for example, magnetic beads carrying such a single stranded oligonucleotide can be used to capture the amplicons and transfer them to a separate vessel containing a nuclease to cleave the tag, e.g. at cleavage site 418. Preferably, the T primer contains a 5' biotin which permits the release tag to be captured and conveniently labeled. After capture, e.g. via avidinated magnetic beads, the 3' strands of the double stranded segment are stripped back to the tag by the use of T4 DNA polymerase, or like enzyme, in the presence of a deoxynucleoside triphosphate (dNTP) corresponding to the nucleotide flanking the tag. Thus, provided that the flanking nucleotides are not present elsewhere along the strand to the 3' ends, the 3'→5' exonuclease activity of the polymerase will strip back the 3' strand to the flanking nucleotides, at which point an exchange reaction will be initiated that prevents further stripping past the flanking nucleotides. The 3' ends of the tag can then be labeled in an extension reaction with labeled dNTPs. After labelling the non-biotinylated strand can be removed by denaturation and applied to the spatially addressable array for detection.

After the labeled tags are hybridized to their tag complements and detected, the tags are removed by washing so that labeled tags from the next set of amplicons can be applied. As long as the amplicon number from which the tags arose are kept track of, the ordering of hybridizations is not crucial. Preferably, hybridizations are done in the same order as the order of the corresponding nucleotide in the target sequence.

The extent to which the S primers can overlap the polynucleotide for identifying successive nucleotides is limited by the degeneracy, or complexity, of the primer mixture as the overlap increases. This difficulty is addressed by periodically cleaving off the identified nucleotides from the polynucleotide, and then starting the identification cycle over again on the shortened polynucleotide. Such cleavage is effected by providing an adaptor with an S primer binding region having a recognition site of a nuclease that has a cleavage site separate from its recognition site. The recognition site of the nuclease is positioned so that it cleaves the polynucleotide a predetermined number of nucleotides from the border of the S primer binding site. Such a nuclease is referred to herein as a "stepping" nuclease. Preferably, a type IIs restriction endonuclease is employed for as a stepping nuclease in the invention. Prior to cleavage the polynucleotide must be treated, e.g. by methylation, to prevent fortuitous cleavage because of internal recognition sites of the stepping nuclease being employed. After cleavage, the strand bearing the S primer binding site is removed, e.g. via triplex capture on magnetic beads, and the adaptor containing a replacement S primer binding site (and stepping nuclease recognition site) is ligated to the remaining strand. The adaptor contains a degenerate protruding strand that is compatible with the cleavage produced by the stepping nuclease. For example, if the type IIs nuclease Bbv I is employed, the adaptor may have the following structure (SEQ ID NO: 7):

```
5'- AAAGAAAGGAAGGGCAGCT
    TTTCTTTCCTTCCCGTCGANNNN
```

where N is defined as above and the underlining indicates the location of a Bbv I recognition site.

A stepping nuclease employed in the invention need not be a single protein, or consist solely of a combination of proteins. A key feature of the stepping nuclease, or of the combination of reagents employed as a stepping nuclease, is that its (their) cleavage site be separate from its (their) recognition site. The distance between the recognition site of a stepping nuclease and its cleavage site will be referred to herein as its "reach." By convention, "reach" is defined by two integers which give the number of nucleotides between the recognition site and the hydrolyzed phosphodiester bonds of each strand. For example, the recognition and cleavage properties of Fok I is typically represented as "GGATG (9/13)" because it recognizes and cuts a double stranded DNA as follows (SEQ ID NO: 8):

5'-... NN**GGATG**NNNNNNNNN     NNNNNNNNNN ...

3'-... NN**CCTAC**NNNNNNNNNNNNN NNNNNN ...

where the bolded nucleotides are Fok I's recognition site and the N's are arbitrary nucleotides and their complements.

Preferably, stepping nucleases employed in the invention are natural protein endonucleases (i) whose recognition site is separate from its cleavage site and (ii) whose cleavage results in a protruding strand on the polynucleotide. Most preferably, type Us restriction endonucleases are employed as stepping nucleases in the invention, e.g. as described in Szybalski et al, Gene, 100: 13-26 (1991); Roberts et al, Nucleic Acids Research, 21: 3125-3137 (1993); and Livak and Brenner, U.S. patent 5,093,245. Exemplary type IIs nucleases include Alw XI, Bsm AI, Bbv I, Bsm FI, Sts I, Hga I, Bsc AI, Bbv II. Bce fI, Bce 85I, Bcc I, Bcg I, Bsa I, Bsg I, Bsp MI, Bst 71 I, Ear I, Eco 57I, Esp 3I, Fau I, Fok I, Gsu I, Hph I, Mbo II, Mme I, Rle AI, Sap I, Sfa NI, Taq II, Tth 111II, Bco 5I, Bpu AI, Fin I, Bsr DI, and isoschizomers thereof. Preferred nucleases include Fok I, Bbv I, Hga I, Ear I, and Sfa NI.

Preferably, prior to nuclease cleavage steps, usually at the start of an identification cycle, the polynucleotide is treated to block the recognition sites and/or cleavage sites of the nuclease being employed. This prevents undesired cleavage of the polynucleotide because of the fortuitous occurrence of nuclease recognition sites at interior locations in the polynucleotide. Blocking can be achieved in a variety of ways, including methylation and treatment by sequence-specific aptamers, DNA binding proteins, or oligonucleotides that form triplexes. Whenever natural protein endonucleases are employed, recognition sites can be conveniently blocked by methylating the polynucleotide with the cognate methylase of the nuclease being used. That is, for most if not all type II bacterial restriction endonucleases, there exists a so-called "cognate" methylases that methylates its recognition site. Many such methylases are disclosed in Roberts et al (cited above) and Nelson et al, Nucleic Acids Research, 21: 3139-3154 (1993), and are commercially available from a variety of sources, particularly New England Biolabs (Beverly, MA).

Oligonucleotide Tags and Tag Complements

In one aspect, the oligonucleotide tags of the invention comprise a plurality of "words" or subunits selected from minimally cross-hybridizing sets of subunits. Subunits of such sets cannot form a duplex or triplex with the complement of another subunit of the same set with less than two mismatched nucleotides. Thus, the sequences of any two oligonucleotide tags of a repertoire that form duplexes will never be "closer" than differing by two nucleotides. In particular embodiments, sequences of any two oligonucleotide tags of a repertoire can be even "further" apart, e.g. by designing a minimally cross-hybridizing set such that subunits cannot form a duplex with the complement of another subunit of the same set with less than three mismatched nucleotides, and so on. Usually, oligonucleotide tags of the invention and their complements are oligomers of the natural nucleotides so that they may be conveniently processed by enzymes, such as ligases, polymerases, nucleases, terminal transferases, and the like.

Complements of oligonucleotide tags of the invention, referred to herein as "tag complements," may comprise natural nucleotides or non-natural nucleotide analogs. Preferably, tag complements are attached to solid phase supports.

Minimally cross-hybridizing sets of oligonucleotide tags and tag complements may be synthesized either combinatorially or individually depending on the size of the set desired and the degree to which cross-hybridization is sought to be minimized (or stated another way, the degree to which specificity is sought to be enhanced). For example, a minimally cross-hybridizing set may consist of a set of individually synthesized 10-mer sequences that differ from each other by at least 4 nucleotides, such set having a maximum size of 332 (when composed of 3 kinds of nucleotides and counted using a computer program such as disclosed in Appendix Ic). Alternatively, a minimally cross-hybridizing set of oligonucleotide tags may also be assembled combinatorially from subunits which themselves are selected from a minimally cross-hybridizing set. For example, a set of minimally cross-hybridizing 12-mers differing from one another by at least three nucleotides may be synthesized by assembling 3 subunits selected from a set of minimally cross-hybridizing 4-mers that each differ from one another by three nucleotides. Such an embodiment gives a maximally sized set of $9^3$, or 729, 12-mers. The number 9 is number of oligonucleotides listed by the computer program of Appendix Ia, which assumes, as with the 10-mers, that only 3 of the 4 different types of nucleotides are used. The set is described as "maximal" because the computer programs of Appendices Ia-c provide the largest set for a given input (e.g. length, composition, difference in number of nucleotides between members). Additional minimally cross-hybridizing sets may be formed from subsets of such calculated sets.

Oligonucleotide tags may be single stranded and be designed for specific hybridization to single stranded tag complements by duplex formation or for specific hybridization to double stranded tag complements by triplex formation. Oligonucleotide tags may also be double stranded and be designed for specific hybridization to single stranded tag complements by triplex formation. Preferably, as used for shuttling sequence information, tags and tag complements are single stranded.

When synthesized combinatorially, an oligonucleotide tag of the invention preferably consists of a plurality of subunits, each subunit consisting of an oligonucleotide of 3 to 9 nucleotides in length wherein each subunit is selected from the same minimally cross-hybridizing set. In such embodiments, the number of oligonucleotide tags available depends on the number of subunits per tag and on the length of the subunits. The number is generally much less than the number of all possible sequences the length of the tag, which for a tag n nucleotides long would be $4^n$.

The nucleotide sequences of oligonucleotides of a minimally cross-hybridizing set are conveniently enumerated by simple computer programs following the general algorithm illustrated in Fig. 1, and as exemplified by programs whose source codes are listed in Appendices Ia and Ib. Program minhx of Appendix Ia computes all minimally cross-hybridizing sets having 4-mer subunits composed of three kinds of nucleotides. Program tagN of Appendix Ib enumerates longer oligonucleotides of a minimally cross-hybridizing set. Similar algorithms and computer programs are readily written for listing oligonucleotides of minimally cross-hybridizing sets for any embodiment of the invention. Table I below provides guidance as to the size of sets of minimally cross-hybridizing oligonucleotides for the indicated lengths and number of nucleotide differences. The above computer programs were used to generate the numbers.

Table I

| Oligonucleotide Word Length | Nucleotide Difference between Oligonucleotides of Minimally Cross-Hybridizing Set | Maximal Size of Minimally Cross-Hybridizing Set | Size of Repertoire with Four Words | Size of Repertoire with Five Words |
|---|---|---|---|---|
| 4 | 3 | 9 | 6561 | $5.90 \times 10^4$ |
| 6 | 3 | 27 | $5.3 \times 10^5$ | $1.43 \times 10^7$ |
| 7 | 4 | 27 | $5.3 \times 10^5$ | $1.43 \times 10^7$ |
| 7 | 5 | 8 | 4096 | $3.28 \times 10^4$ |
| 8 | 3 | 190 | $1.30 \times 10^9$ | $2.48 \times 10^{11}$ |
| 8 | 4 | 62 | $1.48 \times 10^7$ | $9.16 \times 10^8$ |
| 8 | 5 | 18 | $1.05 \times 10^5$ | $1.89 \times 10^6$ |
| 9 | 5 | 39 | $2.31 \times 10^6$ | $9.02 \times 10^7$ |
| 10 | 5 | 332 | $1.21 \times 10^{10}$ | |
| 10 | 6 | 28 | $6.15 \times 10^5$ | $1.72 \times 10^7$ |
| 11 | 5 | 187 | | |
| 18 | 6 | ≈25000 | | |
| 18 | 12 | 24 | | |

For some embodiments of the invention, where extremely large repertoires of tags are not required, oligonucleotide tags of a minimally cross-hybridizing set may be separately synthesized. Sets containing several hundred to several thousands, or even several tens of thousands, of oligonucleotides may be synthesized directly by a variety of parallel synthesis approaches, e.g. as disclosed in Frank et al, U.S. patent 4,689,405; Frank et al, Nucleic Acids Research, 11: 4365-4377 (1983); Matson et al, Anal. Biochem., 224: 110-116 (1995); Fodor et al, International application PCT/US93/04145; Pease et al, Proc. Natl. Acad. Sci., 91: 5022-5026 (1994); Southern et al, J. Biotechnology, 35: 217-227 (1994), Brennan, International application PCTIUS94/05896; Lashkari et al, Proc. Natl. Acad. Sci., 92: 7912-7915 (1995); or the like.

Preferably, oligonucleotide tags of the invention are synthesized combinatorially out of subunits between three and six nucleotides in length and selected from the same minimally cross-hybridizing set. For oligonucletides in this range, the members of such sets may be enumerated by computer programs based on the algorithm of Fig. 1.

The algorithm of Fig. 1 is implemented by first defining the characteristics of the subunits of the minimally cross-hybridizing set, i.e. length, number of base differences between members, and composition, e.g. do they consist of two, three, or four kinds of bases. A table $M_n$, n=1, is generated (100) that consists of all possible sequences of a given length and composition. An initial subunit $S_1$ is selected and compared (120) with successive subunits $S_i$ for i=n+1 to the end of the table. Whenever a successive subunit has the required number of mismatches to be a member of the minimally cross-hybridizing set, it is saved in a new table $M_{n+1}$ (125), that also contains subunits previously selected in prior passes through step 120. For example, in the first set of comparisons, $M_2$ will contain $S_1$; in the second set of comparisons, $M_3$ will contain $S_1$ and $S_2$; in the third set of comparisons, $M_4$ will contain $S_1$, $S_2$, and $S_3$; and so on. Similarly, comparisons in table $M_j$ will be between $S_j$ and all successive subunits in $M_j$. Note that each successive

table $M_{n+1}$ is smaller than its predecessors as subunits are eliminated in successive passes through step 130. After every subunit of table $M_n$ has been compared (140) the old table is replaced by the new table $M_{n+1}$, and the next round of comparisons are begun. The process stops (160) when a table $M_n$ is reached that contains no successive subunits to compare to the selected subunit $S_i$, i.e. $M_n=M_{n+1}$.

Preferably, minimally cross-hybridizing sets comprise subunits that make approximately equivalent contributions to duplex stability as every other subunit in the set. In this way, the stability of perfectly matched duplexes between every subunit and its complement is approximately equal. Guidance for selecting such sets is provided by published techniques for selecting optimal PCR primers and calculating duplex stabilities. e.g. Rychlik et al, Nucleic Acids Research, 17: 8543-8551 (1989) and 18: 6409-6412 (1990); Breslauer et al, Proc. Natl. Acad. Sci., 83: 3746-3750 (1986); Wetmur. Crit. Rev. Biochem. Mol. Biol., 26: 227-259 (1991);and the like. For shorter tags, e.g. about 30 nucleotides or less, the algorithm described by Rychlik and Wetmur is preferred, and for longer tags, e.g. about 30-35 nucleotides or greater, an algorithm disclosed by Suggs et al, pages 683-693 in Brown, editor, ICN-UCLA Symp. Dev. Biol., Vol. 23 (Academic Press, New York, 1981) may be conveniently employed. Clearly, the are many approaches available to one skilled in the art for designing sets of minimally cross-hybridizing subunits within the scope of the invention. For example, to minimize the affects of different base-stacking energies of terminal nucleotides when subunits are assembled, subunits may be provided that have the same terminal nucleotides. In this way, when subunits are linked, the sum of the base-stacking energies of all the adjoining terminal nucleotides will be the same, thereby reducing or eliminating variability in tag melting temperatures.

A "word" of terminal nucleotides, shown in italic below, may also be added to each end of a tag so that a perfect match is always formed between it and a similar terminal "word" on any other tag complement. Such an augmented tag would have the form:

| | | | | | |
|---|---|---|---|---|---|
| *W* | $W_1$ | $W_2$ ... $W_{k-1}$ | $W_k$ | *W* |
| *W'* | $W_1'$ | $W_2'$ ... $W_{k-1}'$ | $W_k'$ | *W'* |

where the primed W's indicate complements. With ends of tags always forming perfectly matched duplexes, all mismatched words will be internal mismatches thereby reducing the stability of tag-complement duplexes that otherwise would have mismatched words at their ends. It is well known that duplexes with internal mismatches are significantly less stable than duplexes with the same mismatch at a terminus.

A preferred embodiment of minimally cross-hybridizing sets are those whose subunits are made up of three of the four natural nucleotides. As will be discussed more fully below, the absence of one type of nucleotide in the oligonucleotide tags permits polynucleotides to be loaded onto solid phase supports by use of the 5'→3' exonuclease activity of a DNA polymerase. The following is an exemplary minimally cross-hybridizing set of subunits each comprising four nucleotides selected from the group consisting of A, G, and T:

**Table II**

| Word: | $w_1$ | $w_2$ | $w_3$ | $w_4$ |
|---|---|---|---|---|
| Sequence: | GATT | TGAT | TAGA | TTTG |
| **Word:** | $w_5$ | $w_6$ | $w_7$ | $w_8$ |
| Sequence: | GTAA | AGTA | ATGT | AAAG |

In this set, each member would form a duplex having three mismatched bases with the complement of every other member.

Further exemplary minimally cross-hybridizing sets are listed below in Table III. Clearly. additional sets can be generated by substituting different groups of nucleotides, or by using subsets of known minimally cross-hybridizing sets.

Table III

Exemplary Minimally Cross-Hybridizing Sets of 4-mer Subunits

| Set 1 | Set 2 | Set 3 | Set 4 | Set 5 | Set 6 |
|---|---|---|---|---|---|
| CATT | ACCC | AAAC | AAAG | AACA | AACG |
| CTAA | AGGG | ACCA | ACCA | ACAC | ACAA |
| TCAT | CACG | AGGG | AGGC | AGGG | AGGC |
| ACTA | CCGA | CACG | CACC | CAAG | CAAC |
| TACA | CGAC | CCGC | CCGG | CCGC | CCGG |
| TTTC | GAGC | CGAA | CGAA | CGCA | CGCA |
| ATCT | GCAG | GAGA | GAGA | GAGA | GAGA |
| AAAC | GGCA | GCAG | GCAC | GCCG | GCCC |
| | AAAA | GGCC | GGCG | GGAC | GGAG |

| Set 7 | Set 8 | Set 9 | Set 10 | Set 11 | Set 12 |
|---|---|---|---|---|---|
| AAGA | AAGC | AAGG | ACAG | ACCG | ACGA |
| ACAC | ACAA | ACAA | AACA | AAAA | AAAC |
| AGCG | AGCG | AGCC | AGGC | AGGC | AGCG |
| CAAG | CAAG | CAAC | CAAC | CACC | CACA |
| CCCA | CCCC | CCCG | CCGA | CCGA | CCAG |
| CGGC | CGGA | CGGA | CGCG | CGAG | CGGC |
| GACC | GACA | GACA | GAGG | GAGG | GAGG |
| GCGG | GCGG | GCGC | GCCC | GCAC | GCCC |
| GGAA | GGAC | GGAG | GGAA | GGCA | GGAA |

The oligonucleotide tags of the invention and their complements are conveniently synthesized on an automated DNA synthesizer, e.g. an Applied Biosystems, Inc. (Foster City, California) model 392 or 394 DNA/RNA Synthesizer, using standard chemistries, such as phosphoramidite chemistry, e.g. disclosed in the following references: Beaucage and Iyer. Tetrahedron, 48: 2223-2311 (1992); Molko et al, U.S. patent 4,980,460; Koster et al, U.S. patent 4,725,677; Caruthers et al, U.S. patents 4,415,732; 4,458,066; and 4,973,679; and the like. Alternative chemistries, e.g. resulting in non-natural backbone groups, such as phosphorothioate, phosphoramidate, and the like, may also be employed provided that the resulting oligonucleotides are capable of specific hybridization. In some embodiments, tags may comprise naturally occurring nucleotides that permit processing or manipulation by enzymes, while the corresponding tag complements may comprise non-natural nucleotide analogs, such as peptide nucleic acids, or like compounds, that promote the formation of more stable duplexes during sorting.

When microparticles are used as supports, repertoires of oligonucleotide tags and tag complements may be generated by subunit-wise synthesis via "split and mix" techniques, e.g. as disclosed in Shortle et al, International patent application PCT/US93/03418 or Lyttle et al, Biotechniques, 19: 274-280 (1995). Briefly, the basic unit of the synthesis is a subunit of the oligonucleotide tag. Preferably, phosphoramidite chemistry is used and 3' phosphoramidite oligonucleotides are prepared for each subunit in a minimally cross-hybridizing set, e.g. for the set first listed above, there would be eight 4-mer 3'-phosphoramidites. Synthesis proceeds as disclosed by Shortle et al or in direct analogy with the techniques employed to generate diverse oligonucleotide libraries using nucleosidic monomers, e.g. as disclosed in Telenius et al, Genomics, 13: 718-725 (1992); Welsh et al, Nucleic Acids Research, 19: 5275-5279 (1991); Grothues et al, Nucleic Acids Research, 21: 1321-1322 (1993); Hartley, European patent application 90304496.4; Lam et al, Nature, 354: 82-84 (1991); Zuckerman et al, Int. J. Pept. Protein Research, 40: 498-507 (1992); and the like. Generally, these techniques simply call for the application of mixtures of the activated monomers to the growing oligonucleotide during the coupling steps. Preferably, oligonucleotide tags and tag complements are synthesized on a DNA synthesizer having a number of synthesis chambers which is greater than or equal to the number of different kinds of words used in the construction of the tags. That is, preferably there is a synthesis chamber corresponding to each type of word. In this embodiment, words are added nucleotide-by-nucleotide, such that if a word consists of five nucleotides there are five monomer couplings in each synthesis chamber. After a word is completely synthesized, the synthesis supports are removed from the chambers, mixed, and redistributed back to the chambers for the next cycle of word addition. This latter embodiment takes advantage of the high coupling yields of monomer addition, e.g. in phosphoramidite chemistries.

Double stranded forms of tags may be made by separately synthesizing the complementary strands followed by mixing under conditions that permit duplex formation. Alternatively, double stranded tags may be formed by first synthesizing a single stranded repertoire linked to a known oligonucleotide sequence that serves as a primer binding site. The second strand is then synthesized by combining the single stranded repertoire with a primer and extending with a polymerase. This latter approach is described in Oliphant et al, Gene, 44: 177-183 (1986). Such duplex tags may then be inserted into cloning vectors along with polynucleotides for sorting and manipulation of the polynucleotide in accordance with the invention.

When tag complements are employed that are made up of nucleotides that have enhanced binding characteristics, such as PNAs or oligonucleotide N3'→P5' phosphoramidates, sorting can be implemented through the formation of D-loops between tags comprising natural nucleotides and their PNA or phosphoramidate complements, as an alternative to the "stripping" reaction employing the 3'→5' exonuclease activity of a DNA polymerase to render a tag single stranded.

Oligonucleotide tags of the invention may range in length from 12 to 60 nucleotides or basepairs. Preferably, oligonucleotide tags range in length from 18 to 40 nucleotides or basepairs. More preferably, oligonucleotide tags range in length from 25 to 40 nucleotides or basepairs. In terms of preferred and more preferred numbers of subunits, these ranges may be expressed as follows:

Table IV

| Numbers of Subunits in Tags in Preferred Embodiments | | | |
|---|---|---|---|
| Monomers in Subunit | Nucleotides in Oligonucleotide Tag | | |
| | (12-60) | (18-40) | (25-40) |
| 3 | 4-20 subunits | 6-13 subunits | 8-13 subunits |
| 4 | 3-15 subunits | 4-10 subunits | 6-10 subunits |
| 5 | 2-12 subunits | 3-8 subunits | 5-8 subunits |
| 6 | 2-10 subunits | 3-6 subunits | 4-6 subunits |

Most preferably, oligonucleotide tags are single stranded and specific hybridization occurs via Watson-Crick pairing with a tag complement.

Preferably, repertoires of single stranded oligonucleotide tags of the invention contain at least 100 members; more preferably, repertoires of such tags contain at least 1000 members; and most preferably, repertoires of such tags contain at least 10,000 members.

Triplex Tags

In embodiments where specific hybridization occurs via triplex formation, coding of tag sequences follows the same principles as for duplex-forming tags; however, there are further constraints on the selection of subunit sequences. Generally, third strand association via Hoogsteen type of binding is most stable along homopyrimidine-homopurine tracks in a double stranded target. Usually, base triplets form in T-A*T or C-G*C motifs (where "-" indicates Watson-Crick pairing and "*" indicates Hoogsteen type of binding); however, other motifs are also possible. For example, Hoogsteen base pairing permits parallel and antiparallel orientations between the third strand (the Hoogsteen strand) and the purine-rich strand of the duplex to which the third strand binds, depending on conditions and the composition of the strands. There is extensive guidance in the literature for selecting appropriate sequences, orientation, conditions, nucleoside type (e.g. whether ribose or deoxyribose nucleosides are employed), base modifications (e.g. methylated cytosine, and the like) in order to maximize, or otherwise regulate, triplex stability as desired in particular embodiments, e.g. Roberts et al, Proc. Natl. Acad. Sci., 88: 9397-9401 (1991); Roberts et al, Science, 258: 1463-1466 (1992); Roberts et al, Proc. Natl. Acad. Sci., 93: 4320-4325 (1996); Distefano et al, Proc. Natl. Acad. Sci., 90: 1179-1183 (1993): Mergny et al, Biochemistry, 30: 9791-9798 (1991); Cheng et al, J. Am. Chem. Soc., 114: 4465-4474(1992); Beal and Dervan, Nucleic Acids Research, 20: 2773-2776 (1992); Beal and Dervan, J. Am. Chem. Soc., 114: 4976-4982 (1992); Giovannangeli et al, Proc. Natl. Acad. Sci., 89: 8631-8635 (1992); Moser and Dervan, Science. 238: 645-650(1987); McShan et al, J. Biol. Chem., 267:5712-5721 (1992); Yoon et al, Proc. Natl. Acad. Sci., 89: 3840-3844 (1992); Blume et al, Nucleic Acids Research, 20: 1777-1784 (1992); Thuong and Helene, Angew. Chem. Int. Ed. Engl. 32: 666-690 (1993); Escude et al, Proc. Natl. Acad. Sci., 93: 4365-4369 (1996); and the like. Conditions for annealing single-stranded or duplex tags to their single-stranded or duplex complements are well known, e.g. Ji et al, Anal. Chem. 65: 1323-1328 (1993); Cantor et al, U.S. patent 5,482,836; and the like. Use of triplex tags has the advantage of not requiring a "stripping" reaction with polymerase to expose the tag for annealing to its complement.

Preferably, oligonucleotide tags of the invention employing triplex hybridization are double stranded DNA and the corresponding tag complements are single stranded. More preferably, 5-methylcytosine is used in place of cytosine in the tag complements in order to broaden the range of pH stability of the triplex formed between a tag and its complement. Preferred conditions for forming triplexes are fully disclosed in the above references. Briefly, hybridization takes place in concentrated salt solution, e.g. 1.0 M NaCl, 1.0 M potassium acetate, or the like, at pH below 5.5 ( or 6.5 if 5-methylcytosine is employed). Hybridization temperature depends on the length and composition of the tag; however, for an 18-20-mer tag of longer, hybridization at room temperature is adequate. Washes may be conducted with less concentrated salt solutions, e.g. 10 mM sodium acetate, 100 mM $MgCl_2$, pH 5.8, at room temperature. Tags may be eluted from their tag complements by incubation in a similar salt solution at pH 9.0.

Minimally cross-hybridizing sets of oligonucleotide tags that form triplexes may be generated by the computer program of Appendix Ic, or similar programs. An exemplary set of double stranded 8-mer words are listed below in capital letters with the corresponding complements in small letters. Each such word differs from each of the other words in the set by three base pairs.

Table V

<u>Exemplary Minimally Cross-Hybridizing</u>

<u>Set of Double Stranded 8-mer Tags</u>

| | | | |
|---|---|---|---|
| 5'-AAGGAGAG<br>3'-TTCCTCTC<br>3'-ttcctctc | 5'-AAAGGGGA<br>3'-TTTCCCCT<br>3'-tttcccct | 5'-AGAGAAGA<br>3'-TCTCTTCT<br>3'-tctcttct | 5'-AGGGGGGG<br>3'-TCCCCCCC<br>3'-tccccccc |
| 5'-AAAAAAAA<br>3'-TTTTTTTT<br>3'-tttttttt | 5'-AAGAGAGA<br>3'-TTCTCTCT<br>3'-ttctctct | 5'-AGGAAAAG<br>3'-TCCTTTTC<br>3'-tccttttc | 5'-GAAAGGAG<br>3'-CTTTCCTC<br>3'-ctttcctc |
| 5'-AAAAAGGG<br>3'-TTTTTCCC<br>3'-tttttccc | 5'-AGAAGAGG<br>3'-TCTTCTCC<br>3'-tcttctcc | 5'-AGGAAGGA<br>3'-TCCTTCCT<br>3'-tccttcct | 5'-GAAGAAGG<br>3'-CTTCTTCC<br>3'-cttcttcc |
| 5'-AAAGGAAG<br>3'-TTTCCTTC<br>3'-tttccttc | 5'-AGAAGGAA<br>3'-TCTTCCTT<br>3'-tcttcctt | 5'-AGGGGAAA<br>3'-TCCCCTTT<br>3'-tcccettt | 5'-GAAGAGAA<br>3'-CTTCTCTT<br>3'-cttctctt |

Table VI

| Repertoire Size of Various Double Stranded Tags That Form Triplexes with Their Tag Complements | | | | |
|---|---|---|---|---|
| Oligonucleotide Word Length | Nucleotide Difference between Oligonucleotides of Minimally Cross-Hybridizing Set | Maximal Size of Minimally Cross-Hybridizing Set | Size of Repertoire with Four Words | Size of Repertoire with Five Words |
| 4 | 2 | 8 | 4096 | $3.2 \times 10^4$ |
| 6 | 3 | 8 | 4096 | $3.2 \times 10^4$ |
| 8 | 3 | 16 | $6.5 \times 10^4$ | $1.05 \times 10^6$ |
| 10 | 5 | 8 | 4096 | |
| 15 | 5 | 92 | | |
| 20 | 6 | 765 | | |
| 20 | 8 | 92 | | |
| 20 | 10 | 22 | | |

Preferably, repertoires of double stranded oligonucleotide tags of the invention contain at least 10 members;

more preferably, repertoires of such tags contain at least 100 members. Preferably, words are between 4 and 8 nucleotides in length for combinatorially synthesized double stranded oligonucletide tags, and oligonucleotide tags are between 12 and 60 base pairs in length. More preferably, such tags are between 18 and 40 base pairs in length.

Solid Phase Supports

Solid phase supports for use with the invention may have a wide variety of forms, including microparticles, beads, and membranes, slides, plates, micromachined chips, and the like. Likewise, solid phase supports of the invention may comprise a wide variety of compositions, including glass, plastic, silicon, alkanethiolate-derivatized gold, cellulose, low cross-linked and high cross-linked polystyrene, silica gel, polyamide, and the like. Preferably, either a population of discrete particles are employed such that each has a uniform coating, or population, of complementary sequences of the same tag (and no other), or a single or a few supports are employed with spatially discrete regions each containing a uniform coating, or population, of complementary sequences to the same tag (and no other). In the latter embodiment, the area of the regions may vary according to particular applications; usually, the regions range in area from several $\mu m^2$, e.g. 3-5, to several hundred $\mu m^2$, e.g. 100-500. Preferably, such regions are spatially discrete so that signals generated by events, e.g. fluorescent emissions, at adjacent regions can be resolved by the detection system being employed. In some applications, it may be desirable to have regions with uniform coatings of more than one tag complement, e.g. for simultaneous sequence analysis, or for bringing separately tagged molecules into close proximity.

Tag complements may be used with the solid phase support that they are synthesized on, or they may be separately synthesized and attached to a solid phase support for use, e.g. as disclosed by Lund et al, Nucleic Acids Research, 16: 10861-10880 (1988); Albretsen et al, Anal. Biochem., 189: 40-50 (1990); Wolf et al, Nucleic Acids Research, 15: 2911-2926 (1987); or Ghosh et al, Nucleic Acids Research, 15: 5353-5372 (1987). Preferably, tag complements are synthesized on and used with the same solid phase support, which may comprise a variety of forms and include a variety of linking moieties. Such supports may comprise microparticles or arrays, or matrices, of regions where uniform populations of tag complements are synthesized. A wide variety of microparticle supports may be used with the invention, including microparticles made of controlled pore glass (CPG), highly cross-linked polystyrene, acrylic copolymers, cellulose, nylon, dextran, latex, polyacrolein, and the like, disclosed in the following exemplary references: Meth. Enzymol., Section A, pages 11-147, vol. 44 (Academic Press, New York, 1976); U.S. patents 4,678,814; 4,413,070; and 4,046;720; and Pon, Chapter 19, in Agrawal, editor, Methods in Molecular Biology, Vol. 20, (Humana Press, Totowa, NJ, 1993). Microparticle supports further include commercially available nucleoside-derivatized CPG and polystyrene beads (e.g. available from Applied Biosystems, Foster City, CA); derivatized magnetic beads; polystyrene grafted with polyethylene glycol (e.g., TentaGel™, Rapp Polymere, Tubingen Germany); and the like. Selection of the support characteristics, such as material, porosity, size, shape, and the like, and the type of linking moiety employed depends on the conditions under which the tags are used. For example, in applications involving successive processing with enzymes, supports and linkers that minimize steric hindrance of the enzymes and that facilitate access to substrate are preferred. Other important factors to be considered in selecting the most appropriate microparticle support include size uniformity, efficiency as a synthesis support, degree to which surface area known, and optical properties, e.g. as explain more fully below, clear smooth beads provide instrumentational advantages when handling large numbers of beads on a surface.

Exemplary linking moieties for attaching and/or synthesizing tags on microparticle surfaces are disclosed in Pon et al, Biotechniques, 6:768-775 (1988); Webb, U.S. patent 4,659,774; Barany et al, International patent application PCT/US91/06103; Brown et al, J. Chem. Soc. Commun., 1989: 891-893; Damha et al, Nucleic Acids Research, 18: 3813-3821 (1990); Beattie et al, Clinical Chemistry, 39: 719-722 (1993); Maskos and Southern, Nucleic Acids Research, 20: 1679-1684 (1992); and the like.

As mentioned above, tag complements may also be synthesized on a single (or a few) solid phase support to form an array of regions uniformly coated with tag complements. That is, within each region in such an array the same tag complement is synthesized. Techniques for synthesizing such arrays are disclosed in McGall et al, International application PCT/US93/03767; Pease et al, Proc. Natl. Acad. Sci., 91: 5022-5026 (1994); Southern and Maskos, International application PCT/GB89/01114; Maskos and Southern (cited above); Southern et al, Genomics, 13: 1008-1017 (1992); and Maskos and Southern, Nucleic Acids Research, 21: 4663-4669 (1993).

Preferably, the invention is implemented with microparticles or beads uniformly coated with complements of the same tag sequence. Microparticle supports and methods of covalently or noncovalently linking oligonucleotides to their surfaces are well known, as exemplified by the following references: Beaucage and Iyer (cited above); Gait, editor, Oligonucleotide Synthesis: A Practical Approach (IRL Press, Oxford, 1984); and the references cited above. Generally, the size and shape of a microparticle is not critical; however, microparticles in the size range of a few, e.g. 1-2, to several hundred, e.g. 200-1000 $\mu m$ diameter are preferable, as they facilitate the construction and manipulation of large repertoires of oligonucleotide tags with minimal reagent and sample usage.

In some preferred applications, commercially available controlled-pore glass (CPG) or polystyrene supports are employed as solid phase supports in the invention. Such supports come available with base-labile linkers and initial nucleosides attached, e.g. Applied Biosystems (Foster City, CA). Preferably, microparticles having pore size between 500 and 1000 angstroms are employed.

In other preferred applications, non-porous microparticles are employed for their optical properties, which may be advantageously used when tracking large numbers of microparticles on planar supports, such as a microscope slide. Particularly preferred non-porous microparticles are the glycidal methacrylate (GMA) beads available from Bangs Laboratories (Carmel, IN). Such microparticles are useful in a variety of sizes and derivatized with a variety of linkage groups for synthesizing tags or tag complements. Preferably, for massively parallel manipulations of tagged microparticles, 5 μm diameter GMA beads are employed.

Attaching Tags to Polynucleotides For Sorting onto Solid Phase Supports

An important aspect of the invention is the sorting and attachment of a populations of polynucleotides, e.g. from a cDNA library, to microparticles or to separate regions on a solid phase support such that each microparticle or region has substantially only one kind of polynucleotide attached. This objective is accomplished by insuring that substantially all different polynucleotides have different tags attached. This condition, in turn, is brought about by taking a sample of the full ensemble of tag-polynucleotide conjugates for analysis. (It is acceptable that identical polynucleotides have different tags, as it merely results in the same polynucleotide being operated on or analyzed twice in two different locations.) Such sampling can be carried out either overtly--for example, by taking a small volume from a larger mixture--after the tags have been attached to the polynucleotides, it can be carried out inherently as a secondary effect of the techniques used to process the polynucleotides and tags, or sampling can be carried out both overtly and as an inherent part of processing steps.

Preferably, in constructing a cDNA library where substantially all different cDNAs have different tags, a tag repertoire is employed whose complexity, or number of distinct tags, greatly exceeds the total number of mRNAs extracted from a cell or tissue sample. Preferably, the complexity of the tag repertoire is at least 10 times that of the polynucleotide population; and more preferably, the complexity of the tag repertoire is at least 100 times that of the polynucleotide population. Below, a protocol is disclosed for cDNA library construction using a primer mixture that contains a full repertoire of exemplary 9-word tags. Such a mixture of tag-containing primers has a complexity of $8^9$, or about $1.34 \times 10^8$. As indicated by Winslow et al, Nucleic Acids Research, 19: 3251-3253 (1991), mRNA for library construction can be extracted from as few as 10-100 mammalian cells. Since a single mammalian cell contains about $5 \times 10^5$ copies of mRNA molecules of about $3.4 \times 10^4$ different kinds, by standard techniques one can isolate the mRNA from about 100 cells, or (theoretically) about $5 \times 10^7$ mRNA molecules. Comparing this number to the complexity of the primer mixture shows that without any additional steps, and even assuming that mRNAs are converted into cDNAs with perfect efficiency (1% efficiency or less is more accurate), the cDNA library construction protocol results in a population containing no more than 37% of the total number of different tags. That is, without any overt sampling step at all, the protocol inherently generates a sample that comprises 37%, or less, of the tag repertoire. The probability of obtaining a double under these conditions is about 5%, which is within the preferred range. With mRNA from 10 cells, the fraction of the tag repertoire sampled is reduced to only 3.7%, even assuming that all the processing steps take place at 100% efficiency. In fact, the efficiencies of the processing steps for constructing cDNA libraries are very low, a "rule of thumb" being that good library should contain about $10^8$ cDNA clones from mRNA extracted from $10^6$ mammalian cells.

Use of larger amounts of mRNA in the above protocol, or for larger amounts of polynucleotides in general, where the number of such molecules exceeds the complexity of the tag repertoire, a tag-polynucleotide conjugate mixture potentially contains every possible pairing of tags and types of mRNA or polynucleotide. In such cases, overt sampling may be implemented by removing a sample volume after a serial dilution of the starting mixture of tag-polynucleotide conjugates. The amount of dilution required depends on the amount of starting material and the efficiencies of the processing steps, which are readily estimated.

If mRNA were extracted from $10^6$ cells (which would correspond to about 0.5 μg of poly(A)$^+$ RNA), and if primers were present in about 10-100 fold concentration excess--as is called for in a typical protocol, e.g. Sambrook et al, Molecular Cloning, Second Edition, page 8.61 [10 μL 1.8 kb mRNA at 1 mg/mL equals about $1.68 \times 10^{-11}$ moles and 10 μL 18-mer primer at 1 mg/mL equals about $1.68 \times 10^{-9}$ moles], then the total number of tag-polynucleotide conjugates in a cDNA library would simply be equal to or less than the starting number of mRNAs, or about $5 \times 10^{11}$ vectors containing tag-polynucleotide conjugates--again this assumes that each step in cDNA construction--first strand synthesis, second strand synthesis, ligation into a vector-- occurs with perfect efficiency, which is a very conservative estimate. The actual number is significantly less.

If a sample of n tag-polynucleotide conjugates are randomly drawn from a reaction mixture--as could be effected by taking a sample volume, the probability of drawing conjugates having the same tag is described by the Poisson distribution, $P(r)=e^{-\lambda}(\lambda)^r/r$, where r is the number of conjugates having the same tag and $\lambda=np$, where p is the probability of a given tag being selected. If $n=10^6$ and $p=1/(1.34 \times 10^8)$, then $\lambda=.00746$ and $P(2)=2.76 \times 10^{-5}$. Thus, a sample of one million molecules gives rise to an expected number of doubles well within the preferred range Such a sample is readily obtained as follows: Assume that the $5 \times 10^{11}$ mRNAs are perfectly converted into $5 \times 10^{11}$ vectors with tag-cDNA conjugates as inserts and that the $5 \times 10^{11}$ vectors are in a reaction solution having a volume of 100 µl. Four 10-fold serial dilutions may be carried out by transferring 10 µl from the original solution into a vessel containing 90 µl of an appropriate buffer, such as TE. This process may be repeated for three additional dilutions to obtain a 100 µl solution containing $5 \times 10^5$ vector molecules per µl. A 2 µl aliquot from this solution yields $10^6$ vectors containing tag-cDNA conjugates as inserts. This sample is then amplified by straight forward transformation of a competent host cell followed by culturing.

Of course, as mentioned above, no step in the above process proceeds with perfect efficiency. In particular, when vectors are employed to amplify a sample of tag-polynucleotide conjugates, the step of transforming a host is very inefficient. Usually, no more than 1% of the vectors are taken up by the host and replicated. Thus, for such a method of amplification, even fewer dilutions would be required to obtain a sample of $10^6$ conjugates.

A repertoire of oligonucleotide tags can be conjugated to a population of polynucleotides in a number of ways, including direct enzymatic ligation, amplification, e.g. via PCR, using primers containing the tag sequences, and the like. The initial ligating step produces a very large population of tag-polynucleotide conjugates such that a single tag is generally attached to many different polynucleotides. However, as noted above, by taking a sufficiently small sample of the conjugates, the probability of obtaining "doubles," i.e. the same tag on two different polynucleotides, can be made negligible. Generally, the larger the sample the greater the probability of obtaining a double. Thus, a design trade-off exists between selecting a large sample of tag-polynucleotide conjugates-which, for example, ensures adequate coverage of a polynucleotide in a shotgun sequencing operation or adequate representation of a rapidly changing mRNA pool, and selecting a small sample which ensures that a minimal number of doubles will be present. In most embodiments, the presence of doubles merely adds an additional source of noise or, in the case of sequencing, a minor complication in scanning and signal processing, as microparticles giving multiple fluorescent signals can simply be ignored.

As used herein, the term "substantially all" in reference to attaching tags to molecules, especially polynucleotides, is meant to reflect the statistical nature of the sampling procedure employed to obtain a population of tag-molecule conjugates essentially free of doubles. The meaning of substantially all in terms of actual percentages of tag-molecule conjugates depends on how the tags are being employed. Preferably, for nucleic acid sequencing, substantially all means that at least eighty percent of the polynucleotides have unique tags attached. More preferably, it means that at least ninety percent of the polynucleotides have unique tags attached. Still more preferably, it means that at least ninety-five percent of the polynucleotides have unique tags attached. And, most preferably, it means that at least ninety-nine percent of the polynucleotides have unique tags attached.

Preferably, when the population of polynucleotides consists of messenger RNA (mRNA), oligonucleotides tags may be attached by reverse transcribing the mRNA with a set of primers preferably containing complements of tag sequences. An exemplary set of such primers could have the following sequence (SEQ ID NO: 9):

```
5'-mRNA- [A]n -3'
          [T]19GG[W,W,W,C]9ACCAGCTGATC-5'-biotin
```

where "$[W,W,W,C]_9$" represents the sequence of an oligonucleotide tag of nine subunits of four nucleotides each and "[W,W,W,C]" represents the subunit sequences listed above, i.e. "W" represents T or A. The underlined sequences identify an optional restriction endonuclease site that can be used to release the polynucleotide from attachment to a solid phase support via the biotin, if one is employed. For the above primer, the complement attached to a microparticle could have the form:

```
5'-[G,W,W,W]9TGG-linker-microparticle
```

After reverse transcription, the mRNA is removed, e.g. by RNase H digestion, and the second strand of the cDNA is synthesized using, for example, a primer of the following form (SEQ ID NO: 10):

5'-NRRGATCYNNN-3'

where N is any one of A, T, G, or C; R is a purine-containing nucleotide, and Y is a pyrimidine-containing nucleotide. This particular primer creates a Bst Y1 restriction site in the resulting double stranded DNA which, together with the Sal I site, facilitates cloning into a vector with, for example, Bam HI and Xho I sites. After Bst Y1 and Sal I digestion, the exemplary conjugate would have the form:

5'-RCGACCA[C,W,W,W]$_9$GG[T]$_{19}$- cDNA -NNNR
GGT[G,W,W,W]$_9$CC[A]$_{19}$- rDNA -NNNYCTAG-5'

The polynucleotide-tag conjugates may then be manipulated using standard molecular biology techniques. For example, the above conjugate--which is actually a mixture-- may be inserted into commercially available cloning vectors, e.g. Stratagene Cloning System (La Jolla, CA); transfected into a host, such as a commercially available host bacteria; which is then cultured to increase the number of conjugates. The cloning vectors may then be isolated using standard techniques, e.g. Sambrook et al, Molecular Cloning, Second Edition (Cold Spring Harbor Laboratory, New York, 1989). Alternatively, appropriate adaptors and primers may be employed so that the conjugate population can be increased by PCR.

Preferably, when the ligase-based method of sequencing is employed, the Bst Y1 and Sal I digested fragments are cloned into a Bam HI-/Xho I-digested vector having the following single-copy restriction sites (SEQ ID NO: 11):

5'-GAGGATGCCTTTATGGATCCACTCGAGATCCCAATCCA-3'
FokI BamHI XhoI

This adds the Fok I site which will allow initiation of the sequencing process discussed more fully below.

Tags can be conjugated to cDNAs of existing libraries by standard cloning methods. cDNAs are excised from their existing vector, isolated, and then ligated into a vector containing a repertoire of tags. Preferably, the tag-containing vector is linearized by cleaving with two restriction enzymes so that the excised cDNAs can be ligated in a predetermined orientation. The concentration of the linearized tag-containing vector is in substantial excess over that of the cDNA inserts so that ligation provides an inherent sampling of tags.

A general method for exposing the single stranded tag after amplification involves digesting a polynucleotide-containing conjugate with the 5'→3' exonuclease activity of T4 DNA polymerase, or a like enzyme. When used in the presence of a single deoxynucleoside triphosphate, such a polymerase will cleave nucleotides from 3' recessed ends present on the non-template strand of a double stranded fragment until a complement of the single deoxynucleoside triphosphate is reached on the template strand. When such a nucleotide is reached the 5'→3' digestion effectively ceases, as the polymerase's extension activity adds nucleotides at a higher rate than the excision activity removes nucleotides. Consequently, single stranded tags constructed with three nucleotides are readily prepared for loading onto solid phase supports.

The technique may also be used to preferentially methylate interior Fok I sites of a polynucleotide while leaving a single Fok I site at the terminus of the polynucleotide unmethylated. First, the terminal Fok I site is rendered single stranded using a polymerase with deoxycytidine triphosphate. The double stranded portion of the fragment is then methylated, after which the single stranded terminus is filled in with a DNA polymerase in the presence of all four nucleoside triphosphates, thereby regenerating the Fok I site. Clearly, this procedure can be generalized to endonucleases other than Fok I.

After the oligonucleotide tags are prepared for specific hybridization, e.g. by rendering them single stranded as described above, the polynucleotides are mixed with microparticles containing the complementary sequences of the tags under conditions that favor the formation of perfectly matched duplexes between the tags and their complements. There is extensive guidance in the literature for creating these conditions. Exemplary references providing such guidance include Wetmur, Critical Reviews in Biochemistry and Molecular Biology, 26: 227-259 (1991); Sambrook et al, Molecular Cloning: A Laboratory Manual, 2nd Edition (Cold Spring Harbor Laboratory, New York, 1989); and the like. Preferably, the hybridization conditions are sufficiently stringent so that only perfectly matched sequences form stable duplexes. Under such conditions the polynucleotides specifically hybridized through their tags may be ligated to the complementary sequences attached to the microparticles. Finally, the microparticles are washed to remove polynucleotides with unligated and/or mismatched tags.

When CPG microparticles conventionally employed as synthesis supports are used, the density of tag complements on the microparticle surface is typically greater than that necessary for some sequencing operations. That is, in sequencing approaches that require successive treatment of the attached polynucleotides with a variety of enzymes, densely spaced polynucleotides may tend to inhibit access of the relatively bulky enzymes to the polynucleotides. In such cases, the polynucleotides are preferably mixed with the microparticles so that tag complements are present in significant excess, e.g. from 10:1 to 100:1, or greater, over the polynucleotides. This ensures that the density of polynucleotides on the microparticle surface will not be so high as to inhibit enzyme access. Preferably, the average inter-polynucleotide spacing on the microparticle surface is on the order of 30-100 nm. Guidance in selecting ratios for standard CPG supports and Ballotini beads (a type of solid glass support) is found in Maskos and Southern, Nucleic Acids Research, 20: 1679-1684 (1992). Preferably, for sequencing applications, standard CPG beads of diameter in the range of 20-50 µm are loaded with about $10^5$ polynucleotides, and GMA beads of diameter in the range of 5-10 µm are loaded with a few tens of thousand polynucleotide, e.g. $4 \times 10^4$ to $6 \times 10^4$.

In the preferred embodiment, tag complements are synthesized on microparticles combinatorially; thus, at the end of the synthesis, one obtains a complex mixture of microparticles from which a sample is taken for loading tagged polynucleotides. The size of the sample of microparticles will depend on several factors, including the size of the repertoire of tag complements, the nature of the apparatus for used for observing loaded microparticles--e.g. its capacity, the tolerance for multiple copies of microparticles with the same tag complement (i.e. "bead doubles"), and the like. The following table provide guidance regarding microparticle sample size, microparticle diameter, and the approximate physical dimensions of a packed array of microparticles of various diameters.

| | | | | |
|---|---|---|---|---|
| Microparticle diameter | 5 µm | 10 µm | 20 µm | 40 µm |
| Max. no. polynucleotides loaded at 1 per $10^5$ sq. angstrom | | $3 \times 10^5$ | $1.26 \times 10^6$ | $5 \times 10^6$ |
| Approx. area of monolayer of $10^6$ microparticles | .45 x .45 cm | 1 x 1 cm | 2 x 2 cm | 4 x 4 cm |

The probability that the sample of microparticles contains a given tag complement or is present in multiple copies is described by the Poisson distribution, as indicated in the following table.

Table VII

| Number of microparticles in sample (as fraction of repertoire size), m | Fraction of repertoire of tag complements present in sample, $1-e^{-m}$ | Fraction of microparticles in sample with unique tag complement attached, m $(e^{-m})/2$ | Fraction of microparticles in sample carrying same tag complement as one other microparticle in sample ("bead doubles"), $m^2(e^{-m})/2$ |
|---|---|---|---|
| 1.000 | 0.63 | 0.37 | 0.18 |
| .693 | 0.50 | 0.35 | 0.12 |
| .405 | 0.33 | 0.27 | 0.05 |
| .285 | 0.25 | 0.21 | 0.03 |
| .223 | 0.20 | 0.18 | 0.02 |
| .105 | 0.10 | 0.09 | 0.005 |
| .010 | 0.01 | 0.01 | |

Apparatus for Observing Detection Signals at Spatially Addressable Sites

Preferably, a spatially addressable array is established by fixing microparticle containing tag complements to a solid phase surface.

Preferably, whenever light-generating signals, e.g. chemiluminescent, fluorescent, or the like, are employed to detect tags, microparticles are spread on a planar substrate, e.g. a glass slide, for examination with a scanning system, such as described in International patent applications PCT/US91/09217, PCT/NL90/00081, and PCT/US95/01886. The scanning system should be able to reproducibly scan the substrate and to define the positions of each microparticle in a predetermined region by way of a coordinate system. In polynucleotide sequencing applications, it is important that the positional identification of microparticles be repeatable in successive scan steps.

Such scanning systems may be constructed from commercially available components, e.g. x-y translation table controlled by a digital computer used with a detection system comprising one or more photomultiplier tubes, or alternatively, a CCD array, and appropriate optics, e.g. for exciting, collecting, and sorting fluorescent signals. In some embodiments a confocal optical system may be desirable. An exemplary scanning system suitable for use in four-color sequencing is illustrated diagrammatically in Figure 3. Substrate 300, e.g. a microscope slide with fixed microparticles, is placed on x-y translation table 302, which is connected to and controlled by an appropriately programmed digital computer 304 which may be any of a variety of commercially available personal computers, e.g. 486-based machines or PowerPC model 7100 or 8100 available form Apple Computer (Cupertino, CA). Computer software for table translation and data collection functions can be provided by commercially available laboratory software, such as Lab Windows, available from National Instruments.

Substrate 300 and table 302 are operationally associated with microscope 306 having one or more objective lenses 308 which are capable of collecting and delivering light to microparticles fixed to substrate 300. Excitation beam 310 from light source 312, which is preferably a laser, is directed to beam splitter 314, e.g. a dichroic mirror, which redirects the beam through microscope 306 and objective lens 308 which, in turn, focuses the beam onto substrate 300. Lens 308 collects fluorescence 316 emitted from the microparticles and directs it through beam splitter 314 to signal distribution optics 318 which, in turn, directs fluorescence to one or more suitable opto-electronic devices for converting some fluorescence characteristic, e.g. intensity, lifetime, or the like, to an electrical signal. Signal distribution optics 318 may comprise a variety of components standard in the art, such as bandpass filters, fiber optics, rotating mirrors, fixed position mirrors and lenses, diffraction gratings, and the like. As illustrated in Figure 5, signal distribution optics 318 directs fluorescence 316 to four separate photomultiplier tubes, 330, 332, 334, and 336, whose output is then directed to pre-amps and photon counters 350, 352, 354, and 356. The output of the photon counters is collected by computer 304, where it can be stored, analyzed, and viewed on video 360. Alternatively, signal distribution optics 318 could be a diffraction grating which directs fluorescent signal 318 onto a CCD array.

The stability and reproducibility of the positional localization in scanning will determine, to a large extent, the resolution for separating closely spaced microparticles. Preferably, the scanning systems should be capable of resolving closely spaced microparticles, e.g. separated by a particle diameter or less. Thus, for most applications, e.g. using CPG microparticles, the scanning system should at least have the capability of resolving objects on the order of 5-100 nm. Even higher resolution may be desirable in some embodiments, but with increase resolution, the time required to fully scan a substrate will increase; thus, in some embodiments a compromise may have to be made between speed and resolution. Increases in scanning time can be achieved by a system which only scans positions where microparticles are known to be located, e.g from an initial full scan. Preferably, microparticle size and scanning system resolution are selected to permit resolution of fluorescently labeled microparticles randomly disposed on a plane at a density between about ten thousand to one hundred thousand microparticles per $cm^2$.

In sequencing applications, microparticles can be fixed to the surface of a substrate in variety of ways. The fixation should be strong enough to allow the microparticles to undergo successive cycles of reagent exposure and washing without significant loss. When the substrate is glass, its surface may be derivatized with an alkylamino linker using commercially available reagents, e.g. Pierce Chemical, which in turn may be cross-linked to avidin, again using conventional chemistries, to form an avidinated surface. Biotin moieties can be introduced to the microparticles in a number of ways.

In an alternative, when DNA-loaded microparticles are applied to a glass substrate, the DNA nonspecifically adsorb to the glass surface upon several hours, e.g. 24 hours, incubation to create a bond sufficiently strong to permit repeated exposures to reagents and washes without significant loss of microparticles. Such a glass substrate may be a flow cell, which may comprise a channel etched in a glass slide. Preferably, such a channel is closed so that fluids may be pumped through it and has a depth sufficiently close to the diameter of the microparticles so that a monolayer of microparticles is trapped within a defined observation region.

**Example 1**

Construction of a Tag Library

An exemplary tag library is constructed as follows to form the chemically synthesized 9-word tags of nucleotides A, G, and T defined by the formula:

$$3'\text{-TGGC-}[^{4}(A,G,T)_{9}]\text{-CCCCp}$$

where "$[^4((A,G,T)_9]$" indicates a tag mixture where each tag consists of nine 4-mer words of A, G, and T; and "p" indicate a 5' phosphate. This mixture is ligated to the following right and left primer binding regions (SEQ ID NO: 12 & 13):

```
5'- AGTGGCTGGGCATCGGACCG        5'- GGGGCCCAGTCAGCGTCGAT
    TCACCGACCCGTAGCCp                    GGGTCAGTCGCAGCTA
```

LEFT RIGHT

The right and left primer binding regions are ligated to the above tag mixture, after which the single stranded portion of the ligated structure is filled with DNA polymerase then mixed with the right and left primers indicated below and amplified to give a tag library.

**Left primer:**

```
5'- AGTGGCTGGGCATCGGACCG

5'- AGTGGCTGGGCATCGGACCG-    [4((A,G,T)9]-   GGGGCCCAGTCAGCGTCGAT
    TCACCGACCCGTAGCCTGGC-    [4((A,G,T)9]-   CCCCGGGTCAGTCGCAGCTA

                                             CCCCGGGTCAGTCGCAGCTA-5'
```

**Right primer**

The underlined portion of the left primer binding region indicates a Rsr II recognition site. The left-most underlined region of the right primer binding region indicates recognition sites for Bsp 120I, Apa I, and Eco O 109I, and a cleavage site for Hga I. The right-most underlined region of the right primer binding region indicates the recognition site for Hga I. Optionally, the right or left primers may be synthesized with a biotin attached (using conventional reagents, e.g. available from Clontech Laboratories, Palo Alto, CA) to facilitate purification after amplification and/or cleavage.

**Example 2**

Construction of a Plasmid Library of Tag-Polynucleotide Conjugates for cDNA "Signature" Sequencing

cDNA is produced from an mRNA sample by conventional protocols using pGGCCCT$_{15}$(A or G or C) as a primer for first strand synthesis anchored at the boundary of the poly A region of the mRNAs and N$_8$(A or T)GATC as the primer for second strand synthesis. That is, both are degenerate primers such that the second strand primer is present in two forms and the first strand primer is present in three forms. The GATC sequence in the second strand primer corresponds to the recognition site of Mbo I; other four base recognition sites could be used as well, such as those for Bam HI, Sph I, Eco RI, or the like. The presence of the A and T adjacent to the restriction site of the second strand primer ensures that a stripping and exchange reaction can be used in the next step to generate a five-base 5' overhang of "GGCCC". The first strand primer is annealed to the mRNA sample and extended with reverse transcriptase, after which the RNA strand is degraded by the RNase H activity of the reverse transcriptase leaving a single stranded cDNA. The second strand primer is annealed and extended with a DNA polymerase using conventional protocols. After second strand synthesis, the resulting cDNAs are methylated with CpG methylase (New England Biolabs, Beverly, MA) using manufacturer's protocols. The 3' strands of the cDNAs are then cut back with the above-mentioned stripping and exchange reaction using T4 DNA polymerase in the presence of dATP and dTTP, after which the cDNAs are ligated to the tag library of Example 1 previously cleaved with Hga I to give the following construct:

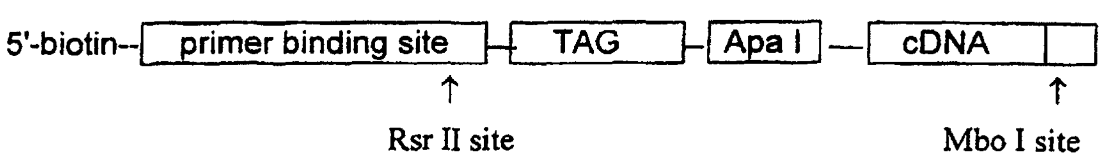


Separately, the following cloning vector is constructed, e.g. starting from a commercially available plasmid, such as a Bluescript phagemid (Stratagene, La Jolla, CA)(SEQ ID NO: 14).

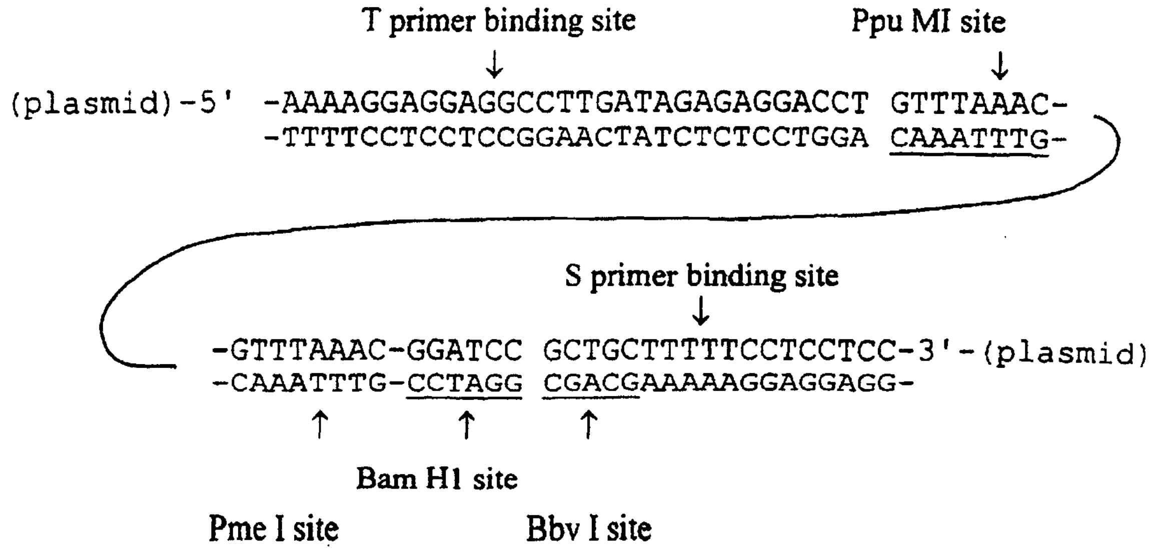


The plasmid is cleaved with Ppu MI and Pme I and then methylated with DAM methylase. The tag-containing construct is cleaved with Rsr II and then ligated to the open plasmid, after which the conjugate is cleaved with Mbo I and Barn HI to permit ligation and closing of the plasmid. The plasmid is then amplified and isolated for use as a template in the selective amplifications using the S and T primers.

**Example 3**

Signature Sequencing of a cDNA Library Using S and T Primers

The plasmid constructed in Example 2 is used as a base library for generating amplicons with S and T primers. The following T primer is employed (SEQ ID NO: 15):

biotin-5'-IIIIIIIAAAAGGAGGAGGCCTTGA

where the I's are deoxyinosines added to balance the annealing and melting temperatures of the S and T primers. The following 32 S primers are employed (SEQ ID NO: 16-23):

XCGACGAAAAAGGAGGAGGIIIIIII-5'
XNCGACGAAAAAGGAGGAGGIIIIII
XNBCGACGAAAAAGGAGGAGGIIIII
XNBBCGACGAAAAAGGAGGAGGIIII
XNBBBCGACGAAAAAGGAGGAGGIII
XNBBBBCGACGAAAAAGGAGGAGGII
XNBBBBBCGACGAAAAAGGAGGAGGI
XNBBBBBBCGACGAAAAAGGAGGAGG

Bbv I site

where X represents one of the four nucleotides, A, C, G, and T so that each of the above sequences represents four different mixtures of S primers, and where N represents a mixture of the four natural nucleotides and B represents a mixture of I and C, which serve as dengeneracy-reducing analogs. Clearly, other spacer nucleotide may also be used.

The plasmid DNA is methylated with Hae III methylase and dispersed into 32 separate vessels, e.g. wells of a microtiter plate, where PCRs using the above primers are performed. Preferably, the reactions are arranged in 8 rows of 4 reactions: one row for each nucleotide position being interrogated and one column for each 3' terminal nucleotide of the S primer. 8 rows are required because the reach of Bbv I is (8/12), so that after 8 nucleotides are identified by the above PCRs, the nucleotides are remove from the cDNA by Bbv I cleavage.

After PCR amplification, the amplicons from each reaction are separately captured on magnetic beads carrying a single stranded sequence that forms a triplex with the S primers. The beads are then transferred to reaction mixtures containing Apa I, which cleaves all strands not containing methyl groups, i.e. all the strands tha have been selectively amplified. The released strands are next captured via their biotinylated T primers with magnetic beads coated with avidin and transferred to reaction vessels where their 3' ends are stripped in the presence of T4 DNA polymerase and dGTP, as shown below:

After cleavage with Apa I (SEQ ID NO: 24):

biotin-5'- IIIIIII$[AG]_{12}$TAGAGAGGACCG[ TAGS ]GGGGCC
CCCCCCCC$[TC]_{12}$ATCTCTCCTGGC[ SGAT ]CC

↓ T4 polymerase + dGTP

biotin-5'- IIIIIII$[AG]_{12}$TAGAGAGGACCG[ TAGS ]GG
GGC[ SGAT ]CC

↓ Add dUTP*, dCTP & ddATP

biotin-5'- IIIIIII$[AG]_{12}$TAGAGAGGACCG[ TAGS ]GG
dAUCUCUCCUGGC[ SGAT ]CC
* * * *

↓ Heat denature

dAUCUCUCCUGGC[ SGAT ]CC-5' (SEQ ID NO 25)
* * * *

Here dUTP* represents a labeled dUTP and ddATP represents dideoxyadenosine triphosphate. Preferably, dUTP is labeled with a separate spectrally resolvable fluorescent dye for each of the four column PCRs. The released tags for each of the eight row PCRs are mixed and are applied to the spatially addressable array for hybridization to their complements and detection.

After, or concurrently with, the 32 PCRs, Bbv I is used to shorten the cDNA inserts of the library. An amplicon is produced from a fresh sample of plasmid using the following S and T primers (SEQ ID NO: 26 & 27):

T primer: 5'-AAAAGGAGGAGGCCTTGA

S primer: 3'-CGACGAAAAAGGAGGAGG-biotin

After amplification, the amplicon is methylated to protect internal Bbv I sites, its 3' ends are stripped using T4 DNA polymerase and dGTP, after which the recessed strands are filled in by the addition of dTTP and dCTP. The amplicon is then cleaved with Bbv I and the S primer segment is removed with magnetic beads coated with avidin. The following adaptor mixture containing a new S primer binding site is then ligated to the T primer segment:

```
5'- GGAGGAGGAAAAAGCAGC
    CCTCCTCCTTTTTCGTCGNNNN
```

The ligated fragment is then amplified for the next cycle of selective amplifications with the 32 S primers described above.

**Example 4**

Signature Sequencing of a cDNA Library Using Rolling Primers

A plasmid library of tag-polynucleotide conjugates is constructed as described in Example 2, except that the primer binding region is constructed as follows:

```
                      T primer binding site                 Ppu MI site
                              ↓                                  ↓
(plasmid)-5' -AAAAGGAGGAGGCCTTGATAGAGAGGACCT GTTTAAAC-
             -TTTTCCTCCTCCGGAACTATCTCTCCTGGA CAAATTTG-

                                     rolling primer binding site
                                              ↓
     -GTTTAAAC-GGATCC-TCTTCCTCTTCCTCTTCC-3'-(plasmid)
     -CAAATTTG-CCTAGG-AGAAGGAGAAGGAGAAGG-
          ↑       ↑
              Bam HI site
      Pme I site
```

The rolling primer binding site corresponds to a rolling primer of subgroup (1), described above. As with Example 2, the above plasmid is cleaved with Ppu MI and Pme I (to give a Rsr II-compatible end and a flush end so that the insert is oriented) and then methylated with DAM methylase. The tag-containing construct (described in Example 2) is then cleaved with Rsr II and then ligated to the open plasmid, after which the conjugate is cleaved with Mbo I and Bam HI to permit ligation and closing of the plasmid. The plasmid is then amplified and isolated for use as a template for extensions and amplifications with rolling primers.

**Example 5**

Signature Sequencing of a cDNA Library with Rolling Primers

The plasmid constructed in Example 2 is used for generating extension products and amplicons with the rolling primers described above and the following T primer (SEQ ID NO: 15):

biotin-5'-IIIIIIIAAAAGGAGGAGGCCTTGA

where the I's are deoxyinosines added to balance the annealing and melting temperatures of the T primers and rolling primers. Preferably, the annealing temperature is about 55°C. Clearly, many other sequences could be employed in the implementation of the invention. The rolling primers described above are employed.

The segment containing the T primer binding site through the rolling primer binding site is excised and separated from the plasmid of example 2. (This can be accomplish in a variety of ways know to those skilled in the art, for example, engineering the plasmid to contain restriction sites flanking the segment, or by simply amplifying directly by PCR). After replacing deoxyguanosines with deoxyinosines, e.g. by PCR in the presence of dITP, the segment is aliquotted into four vessels, denatured, and the appropriate rolling primer is added. Conditions are adjusted to permit the rolling primers to anneal, after which the primers are extended with Sequenase, or like high fidelity polymerase, in the presence of dATP, dCTP, dITP, and dTTP, using the manufacturer's protocol. The remaining single stranded DNA is digested with a single stranded nuclease, such as Mung bean nuclease. Optionally, the double stranded DNA extension product may be separated from the reaction mixture, e.g. by capture via the formation of a triplex between, for example, the T primer binding region, and an appropriate single stranded complement attached to a magnetic bead.

The double stranded DNA is combined with T primer (and rolling primer if a separation step was used) and amplified by 5-10 cycles of PCR in the presence of dATP, dCTP, dITP, and dTTP to form the four initial amplicons. Samples of these are combined and re-distributed into vessels with the appropriate rolling primers for the next cycle of extension. Samples are also drawn off for analysis as described in Example 3.

**APPENDIX Ia**

Exemplary computer program for generating minimally cross hybridizing sets (single stranded tag/single stranded tag complement)

```
Program minxh
c
        integer*2 sub1(6),mset1(1000,6),mset2(1000,6)
        dimension nbase(6)
c
c
        write(*,*)'ENTER SUBUNIT LENGTH'
        read(*,100)nsub
100     format(i1)
        open(1,file='sub4.dat',form='formatted',status='new')
c
c
          nset=0
          do 7000 m1=1,3
             do 7000 m2=1,3
                do 7000 m3=1,3
                   do 7000 m4=1,3
                      sub1(1)=m1
                      sub1(2)=m2
                      sub1(3)=m3
                      sub1(4)=m4
c
        ndiff=3
c
c                  Generate set of subunits differing from
c                  sub1 by at least ndiff nucleotides.
c                  Save in mset1.
c
          jj=1
          do 900 j=1,nsub
900          mset1(1,j)=sub1(j)
c
c
          do 1000 k1=1,3
             do 1000 k2=1,3
                do 1000 k3=1,3
                   do 1000 k4=1,3
c
c
                   nbase(1)=k1
                   nbase(2)=k2
                   nbase(3)=k3
                   nbase(4)=k4
c
                n=0
                do 1200 j=1,nsub
                   if(sub1(j).eq.1 .and. nbase(j).ne.1 .or.
```

```
     1               subl(j).eq.2 .and. nbase(j).ne.2 .or.
     3               subl(j).eq.3 .and. nbase(j).ne.3) then
                     n=n+1
                     endif
 1200                continue
c
c
          if(n.ge.ndiff) then
c
c                                          If number of mismatches
c                                          is greater than or equal
c                                          to ndiff then record
c                                          subunit in matrix mset
c
             jj=jj+1
              do 1100 i=1,nsub
 1100            mset1(jj,i)=nbase(i)
             endif
c
c
 1000     continue
c
c
            do 1325 j2=1,nsub
            mset2(1,j2)=mset1(1,j2)
 1325       mset2(2,j2)=mset1(2,j2)
c
c                                          Compare subunit 2 from
c                                          mset1 with each successive
c                                          subunit in mset1, i.e. 3,
c                                          4,5, ... etc. Save those
c                                          with mismatches .ge. ndiff
c                                          in matrix mset2 starting at
c                                          position 2.
c                                            Next transfer contents
c                                          of mset2 into mset1 and
c                                          start
c                                          comparisons again this time
c                                          starting with subunit 3.
c                                          Continue until all subunits
c                                          undergo the comparisons.
c
          npass=0
c
c
 1700     continue
          kk=npass+2
          npass=npass+1
c
c
          do 1500 m=npass+2,jj
             n=0
             do 1600 j=1,nsub
                if(mset1(npass+1,j).eq.1.and.mset1(m,j).ne.1.or.
     2             mset1(npass+1,j).eq.2.and.mset1(m,j).ne.2.or.
     2             mset1(npass+1,j).eq.3.and.mset1(m,j).ne.3) then
                   n=n+1
```

```
                   endif
1600               continue
                 if(n.ge.ndiff) then
                     kk=kk+1
                     do 1625 i=1,nsub
1625                   mset2(kk,i)=mset1(m,i)
                 endif
1500             continue
c
c                                              kk is the number of subunits
c                                              stored in mset2
c
c                                              Transfer contents of mset2
c                                              into msetl for next pass.
c
c
               do 2000 k=1,kk
                 do 2000 m=1,nsub
2000               msetl(k,m)=mset2(k,m)
           if(kk.lt.jj) then
             jj=kk
             goto 1700
             endif
c
c
            nset=nset+1
           write(1,7009)
7009        format(/)
           do 7008 k=1,kk
7008           write(1,7010)(msetl(k,m),m=1,nsub)
7010       format(4il)
           write(*,*)
           write(*,120) kk,nset
120        format(1x,'Subunits in set=',i5,2x,'Set No=',i5)
7000         continue
            close(1)
c
c
           end
c                    *********************************
c                    *********************************
```

**APPENDIX Ib**

Exemplary computer program for generating minimally cross hybridizing sets (single stranded tag/single stranded tag complement)

```
      Program tagN
c
c
c           Program tagN generates minimally cross-hybridizing
c             sets of subunits given i) N--subunit length, and ii)
c             an initial subunit sequence.  tagN assumes that only
c             3 of the four natural nucleotides are used in the tags.
c
c
        character*1 subl(20)
        integer*2 mset(10000,20), nbase(20)
c
c
        write(*,*)'ENTER SUBUNIT LENGTH'
        read(*,100)nsub
100     format(i2)
c
c
        write(*,*)'ENTER SUBUNIT SEQUENCE'
        read(*,110)(subl(k),k=1,nsub)
110     format(20a1)
c
c
        ndiff=10
c
c
c                Let a=1 c=2 g=3 & t=4
c
c
         do 800 kk=1,nsub
         if(subl(kk).eq.'a') then
            mset(1,kk)=1
            endif
                 if(subl(kk).eq.'c') then
                    mset(1,kk)=2
                    endif
                         if(subl(kk).eq.'g') then
                            mset(1,kk)=3
                            endif
                                 if(subl(kk).eq.'t') then
                                    mset(1,kk)=4
                                    endif
800     continue
c
c
c                Generate set of subunits differing from
c                subl by at least ndiff nucleotides.
c
c
         jj=1
c
c
         do 1000 kl=1,3
```

```
              do 1000 k2=1,3
                do 1000 k3=1,3
                  do 1000 k4=1,3
                    do 1000 k5=1,3
                      do 1000 k6=1,3
                        do 1000 k7=1,3
                          do 1000 k8=1,3
                            do 1000 k9=1,3
                              do 1000 k10=1,3
             do 1000 k11=1,3
               do 1000 k12=1,3
                 do 1000 k13=1,3
                   do 1000 k14=1,3
                     do 1000 k15=1,3
                       do 1000 k16=1,3
                         do 1000 k17=1,3
                           do 1000 k18=1,3
                             do 1000 k19=1,3
                               do 1000 k20=1,3
c
c
                     nbase(1)=k1
                     nbase(2)=k2
                     nbase(3)=k3
                     nbase(4)=k4
                     nbase(5)=k5
                     nbase(6)=k6
                     nbase(7)=k7
                     nbase(8)=k8
                     nbase(9)=k9
                     nbase(10)=k10
                     nbase(11)=k11
                     nbase(12)=k12
                     nbase(13)=k13
                     nbase(14)=k14
                     nbase(15)=k15
                     nbase(16)=k16
                     nbase(17)=k17
                     nbase(18)=k18
                     nbase(19)=k19
                     nbase(20)=k20
c
c
                 do 1250 nn=1,jj
c
                  n=0
                  do 1200 j=1,nsub
                     if(mset(nn,j).eq.1 .and. nbase(j).ne.1 .or.
     1                  mset(nn,j).eq.2 .and. nbase(j).ne.2 .or.
     2                  mset(nn,j).eq.3 .and. nbase(j).ne.3 .or.
     3                  mset(nn,j).eq.4 .and. nbase(j).ne.4) then
                        n=n+1
                        endif
1200                    continue
c
c
           if(n.lt.ndiff) then
              goto 1000
              endif
1250       continue
c
c
               jj=jj+1
               write(*,130)(nbase(i),i=1,nsub),jj
                do 1100 i=1,nsub
```

```
                  mset(jj,i)=nbase(i)
1100                 continue
c
c
1000      continue
c
c
          write(*,*)
130       format(10x,20(1x,i1),5x,i5)
          write(*,*)
          write(*,120) jj
120       format(1x,'Number of words=',i5)
c
c
          end
c
c               **********************************************
c               **********************************************
```

**APPENDIX Ic**

Exemplary computer program for generating minimally cross hybridizing sets (double stranded tag/single stranded tag complement)

```
Program 3tagN
c
c          Program 3tagN generates minimally cross-hybridizing
c            sets of duplex subunits given i) N--subunit length,
c            and ii) an initial homopurine sequence.
c
        character*1 sub1(20)
        integer*2 mset(10000,20), nbase(20)
c
        write(*,*)'ENTER SUBUNIT LENGTH'
        read(*,100)nsub
100     format(i2)
c
        write(*,*)'ENTER SUBUNIT SEQUENCE a & g only'
        read(*,110)(sub1(k),k=1,nsub)
110     format(20a1)
c
        ndiff=10
c
c                 Let a=1 and g=2
c
          do 800 kk=1,nsub
          if(sub1(kk).eq.'a') then
             mset(1,kk)=1
             endif
                  if(sub1(kk).eq.'g') then
                     mset(1,kk)=2
                     endif
800     continue
c

          jj=1
c
          do 1000 k1=1,3
            do 1000 k2=1,3
              do 1000 k3=1,3
                do 1000 k4=1,3
                  do 1000 k5=1,3
                    do 1000 k6=1,3
                      do 1000 k7=1,3
                        do 1000 k8=1,3
                          do 1000 k9=1,3
                            do 1000 k10=1,3
           do 1000 k11=1,3
             do 1000 k12=1,3
               do 1000 k13=1,3
                 do 1000 k14=1,3
                   do 1000 k15=1,3
                     do 1000 k16=1,3
                       do 1000 k17=1,3
                         do 1000 k18=1,3
                           do 1000 k19=1,3
                             do 1000 k20=1,3
c
c
                  nbase(1)=k1
```

```
                  nbase(2)=k2
                  nbase(3)=k3
                  nbase(4)=k4
                  nbase(5)=k5
                  nbase(6)=k6
                  nbase(7)=k7
                  nbase(8)=k8
                  nbase(9)=k9
                  nbase(10)=k10
                  nbase(11)=k11
                  nbase(12)=k12
                  nbase(13)=k13
                  nbase(14)=k14
                  nbase(15)=k15
                  nbase(16)=k16
                  nbase(17)=k17
                  nbase(18)=k18
                  nbase(19)=k19
                  nbase(20)=k20
c
c
              do 1250 nn=1,jj
c
               n=0
               do 1200 j=1,nsub
                  if(mset(nn,j).eq.1 .and. nbase(j).ne.1 .or.
     1               mset(nn,j).eq.2 .and. nbase(j).ne.2 .or.
     2               mset(nn,j).eq.3 .and. nbase(j).ne.3 .or.
     3               mset(nn,j).eq.4 .and. nbase(j).ne.4) then
                     n=n+1
                     endif
1200                 continue
c
c
          if(n.lt.ndiff) then
             goto 1000
             endif
1250      continue
c
               jj=jj+1
               write(*,130)(nbase(i),i=1,nsub),jj
                do 1100 i=1,nsub
                   mset(jj,i)=nbase(i)
1100                  continue
c
1000      continue
c
           write(*,*)
130        format(10x,20(1x,i1),5x,i5)
           write(*,*)
           write(*,120) jj
120        format(1x,'Number of words=',i5)
c
c
           end
```

## Claims

**1.** A method for simultaneously identifying one or more terminal nucleotides of polynucleotides in a population of polynucleotides, the method comprising the steps of:

(a) attaching an oligonucleotide tag from a repertoire of tags to a terminus of each polynucleotide of the popularion. to form tag-polynucleotide conjugates, such that substantially all different polynucleotides have different oligonucleotide tags attached, the oligonucleotide tags being selected from the same minimally cross hybridizing set;

(b) labeling each oligonucleotide tag, or a copy thereof, such that the label identifies one or more terminal nucleotides of the polynucleotide in the tag-polynucleotide conjugate, and, prior to or following such labeling, cleaving the tag or copy thereof from the attached polynucleotide;
(c) transferring the cleaved oligonucleotide tags or copies thereof to a spatially addressable array of tag complements, such that the oligonucleotide tags or copies thereof specifically hybridize to their respective tag complements; and
(d) detecting the labels of the oligonucleotide tags or copies thereof on the spatially addressable array for the identification of the one or more terminal nucleotides of the polynucleotides in the population.

**2.** The method of claim 1, wherein said population of polynucleotides is a population of mRNA molecules, and wherein said population of polynucleotides is formed by:

forming a population of cDNA molecules from the population of mRNA molecules such that each cDNA molecule has an oligonucleotide tag attached at a terminus, the oligonucleotide tags being selected from the same minimally cross hybridising set; and
removing a sample of cDNA molecules from the population such that substantially all different cDNA molecules in the sample have different oligonucleotide tags attached;
and said method further comprises the steps off

(e) shortening the cDNA molecules by removing the identified one or more terminal nucleotides;
(f) repeating steps (b) through (e) until a portion of the sequence of each cDNA molecule is determined; and
(g) identifying the population of mRNA molecules by a frequency distribution of the portions of sequences of the cDNA molecules.

**3.** The method of claim 1, further including the steps of (e) shortening said polynucleotides; and (f) repeating steps (b) through (e).

**4.** The method of any one of claims 1 to 3, wherein said tag-polynucleotide conjugates further comprise first and second primer binding sites, and said step of labelling includes
selectively amplifying said tag-polynucleotide conjugates by a polymerase chain reaction, using a first primer and a second primer, the second primer having a defined 3' terminal nucleotide and forming a duplex with said second primer binding site and one or more terminal nucleotides at one end of the polynucleotide,
such that a tag-polynucleotide conjugate is amplified only if the defined 3' terminal nucleotide basepairs with one of the one or more terminal nucleotides at the end of the polynucleotide, and
labeling tags of amplified conjugates in accordance with the identity of said defined 3' terminal nucleotide.

**5.** The method of any one of claims 2 to 4, wherein said step of shortening includes
providing a nuclease and a nuclease recognition site in said duplex formed between said second primer and said primer binding site, the nuclease having a recognition site separate from its cleavage site, and the recognition site being positioned to permit cleavage of said polynucleotide, such that said one or more terminal nucleotides at said end of the polynucleotide are removed, and
cleaving said terminal nucleotides from the polynucleotide using said nuclease.

**6.** The method of any one of claims 3 to 5, wherein said step of shortening further includes
ligating an adaptor to said end of said polynucleotide after said cleaving, the adaptor containing said second primer binding site and said nuclease recognition site.

**7.** The method of claim 6, wherein said oligonucleotide tag is single stranded and consists of a plurality of subunits, each subunit consisting of an oligonucleotide of 3 to 9 nucleotides in length and each subunit being selected from the same minimally cross-hybridizing set.

**8.** The method of claim 7, wherein said repertoire of said oligonucleotide tags contains at least 100 of said oligonucleotide tags.

**9.** The method of claim 8, wherein said subunits of said oligonucleotide tags are oligonucleotides each having a length between 4 and 9 nucleotides, and wherein each of said oligonucleotide tag differs from every other oligonucleotide tag of said same minimally cross-hybridizing set by at least three nucleotides.

**10.** The method of any one of claims 1 to 4, wherein said step of providing said label includes

(i) providing a set of primers, each primer of the set having a terminal nucleotide, an extension region comprising one or more complexity-reducing nucleotides or complements thereof, and a template positioning segment,
(ii) forming a plurality of templates, each comprising, a primer binding site and a tag-polynucleotide conjugate, the primer binding site being complementary to at least one primer of the set;
(iii) extending primers whose extension regions form a perfectly matched duplex with the primer binding site of a template, to form double stranded DNAs;
(iv) forming amplicons from the templates by amplifying the double stranded DNAs formed by such extending, and
(v) labeling the tags in said amplicons in accordance with the terminal nucleotide of the primer used in said extension and amplification.

**11.** , The method of claim 10, wherein said step of shortening said polynucleotides comprises
mutating said primer binding site of said template, by extending and amplifying each of said templates with a primer whose said template positioning segment contains a nucleotide mismatched with its adjacent nucleotide in said primer binding site of said template, such that the identity of the adjacent nucleotide is changed in said amplicons by oligonucleotide-directed mutagenesis using said primer.

**12.** The method of claim 11, wherein said complexity-reducing nucleotide is deoxyinosine and wherein said polymerase chain reaction and said extending to form said double stranded DNA are carried out in the presence of deoxyadenosine triphosphate, deoxycytidine triphosphate, deoxyinosine triphosphate, and thymidine triphosphate.

## Patentansprüche

**1.** Verfahren zur gleichzeitigen Identifizierung eines oder mehrerer terminalen Nucleotids/Nucleotide von Polynucleotiden in einer Population von Polynucleotiden, wobei das Verfahren die Schritte umfasst:

(a) Anfügen eines Oligonucleotid-Anhangs (Tag) aus einem Repertoire von Anhängen (Tags) an ein Ende eines jeden Polynucleotids der Population, um Anhang(Tag)-Polynucleotid-Konjugate zu bilden, so dass im Wesentlichen alle verschiedenen Polynucleotide verschiedene Oligonucleotid-Anhänge (Tags) angefügt haben, wobei die Oligonucleotid-Anhänge (Tags) ausgewählt sind aus dem gleichen minimal kreuzhybridisierenden Satz;
(b) Markieren eines jeden Oligonucleotid-Anhangs (Tag) oder einer Kopie davon, so dass die Markierung ein oder mehrere terminale(s) Nucleotid(e) der Polynucleotide in dem Anhang(Tag)-Polynucleotid-Konjugate identifiziert und, vor oder nach einem solchen Markieren, Abspalten des Anhangs (Tags) oder einer Kopie davon von dem angefügten Polynucleotid;
(c) Überführen der abspaltenen Oligonucleotid-Anhänge (Tags) oder Kopien davon auf eine räumlich zuordenbare Anordnung von Anhang (Tag)-Komplementen, so dass die Oligonucleotid-Anhänge (Tags) oder Kopien davon spezifisch an ihre entsprechenden Anhang(Tag)-Komplemente hybridisieren; und
(d) Nachweisen der Markierungen der Oligonucleotid-Anhänge (Tags) oder Kopien davon auf der räumlich zuordenbaren Anordnung zur Identifizierung eines oder mehrerer terminalen Nucleotids/Nucleotide der Polynucleotide in der Population.

**2.** Verfahren nach Anspruch 1, wobei die Population der Polynucleotide eine Population von mRNA-Molekülen ist, und wobei die Population der Polynucleotide gebildet wird durch:

Bilden einer Population von cDNA-Molekülen aus der Population von mRNA-Molekülen, so dass jedes cDNA-Molekül einen Oligonucleotid-Anhang (Tag) an einem Ende angefügt hat, wobei die Oligonucleotid-Anhänge (Tags) ausgewählt sind aus dem gleichen minimal kreuzhybridisierenden Satz; und Entnehmen einer Probe von cDNA-Molekülen aus der Population, so dass im Wesentlichen alle verschiedenen cDNA-Moleküle in der Probe verschiedene Oligonucleotid-Anhänge (Tags) angefügt haben; und wobei das Verfahren weiterhin die Schritte umfasst:

(e) Verkürzen der cDNA-Moleküle durch Entfernen von einem oder mehreren identifizierten terminalen Nucleotid(en);

(f) Wiederholen der Schritte (b) bis (e), bis ein Teil der Sequenz eines jeden cDNA-Moleküls bestimmt ist; und
(g) Identifizieren der Population von mRNA-Molekülen durch Häufigkeitsverteilung der Anteile von Sequenzen der cDNA-Moleküle.

**3.** Verfahren nach Anspruch 1, zusätzlich beinhaltend die Schritte: (e) Verkürzen der Polynucleotide; und (f) Wiederholen der Schritte (b) bis (e).

**4.** Verfahren nach einem der Ansprüche 1 bis 3, wobei die Anhang(Tag)-Polynucleotid-Konjugate zusätzlich eine erste und zweite Primer-Bindestelle umfassen und der Schritt des Markierens selektives Amplifizieren der Anhang (Tag)-Polynucleotid-Konjugate durch eine Polymerasekettenreaktion einschließt, durch Verwenden eines ersten Primers und eines zweiten Primers, wobei der zweite Primer ein definiertes 3'-terminales Nucleotid besitzt und ein Duplex mit der zweiten Primer-Bindestelle und einem oder mehreren terminalen Nucleotid(en) an einem Ende des Polynucleotids bildet,
so dass ein Anhang(Tag)-Polynucleotid-Konjugat nur dann amplifiziert wird, wenn das definierte 3'-terminale Nucleotid mit dem einen oder einem von mehreren terminalen Nucleotid/Nucleotiden am Ende des Polynucleotids eine Basenpaarung eingeht und
Markieren der Anhänge (Tags) der amplifizierten Konjugate gemäß der Identität des definierten 3'-terminalen Nucleotids.

**5.** Verfahren nach einem der Ansprüche 2 bis 4, wobei der Schritt des Verkürzens beinhaltet:

Bereitstellen einer Nuclease und einer Nuclease-Erkennungsstelle in dem Duplex, der zwischen dem zweiten Primer und der Primer-Bindestelle gebildet wurde, wobei die Nuclease eine Erkennungsstelle hat, die getrennt ist von ihrer Schnittstelle, und die Erkennungsstelle so gelegen ist, dass sie das Schneiden des Polynucleotides zulässt, so dass das eine oder die mehreren terminale(n) Nucleotid(e) am Ende des Polynucleotids entfernt werden, und Abtrennen der terminalen Nucleotide von dem Polynucleotid durch Verwenden der Nuclease.

**6.** Verfahren nach einem der Ansprüche 3 bis 5, wobei der Schritt des Verkürzens zusätzlich das Ligieren eines Adaptors an das Ende des Polynucleotids nach dem Schneiden einschließt, wobei der Adaptor die zweite Primer-Bindestelle und die Nuclease-Erkennungsstelle enthält.

**7.** Verfahren nach Anspruch 6, wobei der Oligonucleotid-Anhang (Tag) einzelsträngig ist und aus einer Vielzahl von Untereinheiten besteht, wobei jede Untereinheit aus einem Oligonucleotid von 3 bis 9 Nucleotiden in der Länge besteht und jede Untereinheit ausgewählt ist aus dem gleichen minimal kreuzhybridisierenden Satz.

**8.** Verfahren nach Anspruch 7, wobei das Repertoire der Oligonucleotid-Anhänge (Tags) mindestens 100 der Oligonucleotid-Anhänge (Tags) beinhaltet.

**9.** Verfahren nach Anspruch 8, wobei die Untereinheiten der Oligonucleotid-Anhänge (Tags) Oligonucleotide sind, von denen jedes eine Länge zwischen 4 und 9 Nucleotiden hat und wobei jeder Oligonucleotid-Anhang (Tag) sich von jedem anderen Oligonucleotid-Anhang (Tag) des gleichen minimal kreuzhybridisierenden Satzes durch mindestens 3 Nucleotide unterscheidet.

**10.** Verfahren nach einem der Ansprüche 1 bis 4, wobei der Schritt des Bereitstellens der Markierung einschließt:

(i) Bereitstellen eines Satzes von Primern, wobei jeder Primer des Satzes ein terminales Nucleotid, einen Verlängerungsbereich, umfassend ein oder mehrere Komplexität-reduzierende(s) Nucleotid(e) oder Komplemente davon, und ein Matrizen-positionierendes Segment hat,
(ii) Bilden einer Vielzahl von Matrizen, von denen jede eine Primer-Bindestelle und ein Anhang(Tag)-Polynucleotid-Konjugat umfasst, wobei die Primer-Bindestelle komplementär zu mindestens einem Primer des Satzes ist;
(iii) Verlängern der Primer, deren Verlängerungsbereiche einen perfekt passenden Duplex mit der Primer-Bindestelle der Matrize ausbilden, um doppelsträngige DNAs zu bilden;
(iv) Bilden von Amplicons von den Matrizen durch Amplifizieren von doppelsträngigen DNAs, die durch Verlängern gebildet werden, und
(v) Markieren der Anhänge (Tags) in den Amplicons gemäß dem terminalen Nucleotid des Primers, der bei der Verlängerung und Amplifizierung benutzt wurde.

**11.** Verfahren nach Anspruch 10, wobei der Schritt des Verkürzens der Polynucleotide umfasst:

Mutieren der Primer-Bindestelle der Matrize durch Verlängern und Amplifizieren von jeder der Matrizen mit einem Primer, dessen Matrizepositionierendes Segment ein fehlgepaartes Nucleotid mit seinem angrenzenden Nucleotid in der Primer-Bindestelle der Matrize beinhaltet, so dass die Identität des angrenzenden Nucleotids in dem Amplicon durch Oligonucleotid-gerichtete Mutagenese durch Verwenden des Primers verändert wird.

**12.** Verfahren nach Anspruch 11, wobei das Komplexität-reduzierende Nucleotid Desoxyinosin ist und wobei die Polymerasekettenreaktion und das Verlängern, um die doppelsträngige DNA zu bilden, in Anwesenheit von Desoxyadenosintriphosphat, Desoxycytidintriphosphat, Desoxyinosintriphosphat und Thymidintriphosphat durchgeführt wird.

## Revendications

**1.** Méthode pour identifier simultanément un ou plusieurs nucléotides terminaux de polynucléotides dans une population de polynucléotides, la méthode comprenant les étapes de :

(a) attachement d'une étiquette oligonucléotidique provenant d'un répertoire d'étiquettes à une extrémité de chaque polynucléotide de la population pour former des conjugés polynucléotide-étiquettes, de manière à ce que practiquement tous les polynucléotides différents aient des étiquettes oligonucléotidiques différentes qui leur sont attachées, les étiquettes oligonucléotidiques étant choisies dan le même ensemble susceptible d'hybridation croisée minimale ;
(b) marquage de chaque étiquette oligonucléotidique, ou d'une copie de celle-ci, de manière que le marqueur identifie un ou plusieurs nucléotides terminaux du polynucléotide du conjugué étiquette-polynucléotide, et, avant ou après ce marquage, le clivage de l'étiquette ou de sa copie du polynucléotide qui y est attaché ;
(c) transfert des étiquettes oligonucléotidiques ou de leurs copies clivées à un réseau de compléments des étiquettes de positions spatiales déterminables, de manière à ce que les étiquettes oligonucléotidiques ou leurs copies s'hybrident de façon spécifique à leurs compléments d'étiquettes respectifs ; et
(d) détection des marqueurs des étiquettes oligonucléotidiques ou de leurs copies sur le réseau de positions spatiales déterminables en vue de l'identification d'un ou plusieurs nucléotides terminaux des polynucléotides de la population.

**2.** Méthode selon la revendication 1, dans laquelle ladite population de polynucléotides est une population de molécules d'ARNm, et dans laquelle ladite population de polynucléotides est formée par :

formation d'une population de molécules d'ADNc à partir de la population de molécules d'ARNm de manière à ce que chaque molécule d'ADNc ait une étiquette oligonucléotidique attachée à une extrémité , les étiquettes oligonucléotidiques étant choisies dans le même ensemble susceptible d'hybridation croisée minimale ; et retrait d'un échantillon de molécules d'ADNc de la population de sorte que pratiquement toutes les molécules différentes d'ADNc de l'échantillon aient des étiquettes oligonucléotidiques différentes qui leur sont attachées ; et ladite méthode comprenant en outre les étapes de :

(e) raccourcissement des molécules d'ADNc par enlèvement des un ou plusieurs nucléotides terminaux identifiés ;
(f) répétition des étapes (b) à (e) jusqu'à ce que une partie de la séquence de chaque molécule d'ADNc soit déterminée ; et
(g) identification de la population de molécules d'ARNm par une distribution de fréquence des parties de séquences des molécules d'ADNc.

**3.** Méthode selon la revendication 1, comprenant en outre les étapes de (e) raccourcissement desdits polynucléotides ; et (f) répétition des étapes (b) à (e).

**4.** Méthode selon l'une quelconque des revendications 1 à 3, dans laquelle lesdits conjugués étiquette-polynucléotides comprennent en outre un premier et un second site de liaison d'amorce , et ladite étape de marquage inclut

l'amplification sélective des conjugués étiquette-polynucléotides par une réaction de polymérase en chaîne, à l'aide d'une première et d'une seconde amorces, la seconde amorce ayant un nucléotide terminal 3'défini et formant un duplex avec ledit second site de liaison d'amorce et un ou plusieurs nucléotides terminaux à une extrémité du polynucléotide,

de sorte qu'un conjugué étiquette-polynucléotide est amplifié seulement si le nucléotide terminal 3' défini s'apparie avec l'un des un ou plusieurs nucléotides terminaux à l'extrémité du polynucléotide, et

le marquage des étiquettes des conjugués amplifiés en conformité avec l'identité dudit nucléotide terminal 3' défini.

**5.** Méthode selon l'une quelconque des revendications 2 à 4, dans laquelle ladite étape de raccourcissement inclut

la fourniture d'une nucléase et d'un site de reconnaissance par une nucléase dans ledit duplex formé entre ladite seconde amorce et ledit site de liaison d'amorce, la nucléase ayant un site de reconnaissance distinct de son site de clivage, et le site de reconnaissance étant positionné de manière à permettre le clivage dudit polynucléotide, de manière à ce que lesdits un ou plusieurs nucléotides terminaux à ladite extrémité du polynucléotide soient éliminés, et

le clivage desdits nucléotides terminaux du polynucléotide à l'aide de ladite nucléase.

**6.** Méthode selon l'une quelconque des revendications 3 à 5, dans laquelle ladite étape de raccourcissement inclut en outre

la ligation d'un adaptateur à ladite extrémité dudit polynucléotide après ledit clivage, l'adaptateur contenant ledit second site de liaison d'amorce et ledit site de reconnaissance de la nucléase.

**7.** Méthode selon la revendication 6, dans laquelle ladite étiquette oligonucléotidique est simple brin et consiste en une pluralité de sous-unités , chaque sous-unité consistant en un oligonucléotide de 3 à 9 nucléotides de longueur et chaque sous-unité étant choisie dans le même ensemble susceptible d'hybridation croisée minimale.

**8.** Méthode selon la revendication 7, dans laquelle ledit répertoire desdites étiquettes oligonucléotidiques contient au moins 100 desdites étiquettes oligonucléotidiques.

**9.** Méthode selon la revendication 8, dans laquelle lesdites sous-unités desdites étiquettes oligonucléotidiques sont des oligonucléotides ayant chacun une longueur entre 4 et 9 nucléotides, et dans laquelle chacune desdites étiquettes oligonucléotidiques diffère de toutes les autres étiquettes oligonucléotidiques dudit même ensemble susceptible d'hybridation croisée minimale par au moins trois nucléotides.

**10.** Méthode selon l'une quelconque des revendications 1 à 4, dans laquelle ladite étape de fourniture dudit marqueur inclut

(i) la fourniture d'un jeu d'amorces, chaque amorce du jeu ayant un nucléotide terminal, une région d'extension comprenant un ou plusieurs nucléotides de réduction de complexité ou des compléments de ceux-ci, et un segment de positionnement d'une matrice,
(ii) la formation d'une pluralité de matrices, chacune comprenant un site de liaison d'amorce et un conjugué étiquette-polynucléotide, le site de liaison d'amorce étant complémentaire d'au moins une amorce du jeu ;
(iii) l'extension des amorces dont les régions d'extension forment un duplex de complémentarité parfaite avec le site de liaison d'amorce d'une matrice, pour former des ADN double-brin ;
(iv) la formation d'amplicons à partir des matrices par amplification des ADN double- brin formés par cette extension, et
(v) le marquage des étiquettes dans lesdits amplicons en conformité avec le nucléotide terminal de l'amorce utilisée dans lesdites extension et amplification.

**11.** Méthode selon la revendication 10, dans laquelle ladite étape de raccourcissement desdits polynucléotides comprend :

la mutation dudit site de liaison d'amorce de ladite matrice, par extension et amplification de chacune desdites matrices avec une amorce dont le segment de positionnement de la matrice contient un nucléotide mésapparié par rapport à son nucléotide adjacent dans ledit site de liaison d'amorce de ladite matrice, de manière à ce que l'identité du nucléotide adjacent soit modifiée dans lesdits amplicons par mutagénèse dirigée par oligonucléotide à l'aide de ladite amorce.

**12.** Méthode selon la revendication 11, dans laquelle ledit nucléotide de réduction de complexité est la déoxyinosine et dans laquelle ladite réaction de polymérase en chaîne et ladite extension pour former ledit ADN double-brin sont effectuées en présence de déoxyadénosine triphosphate, déoxycytidine triphosphate, déoxyinosine triphosphate et thymidine triphosphate

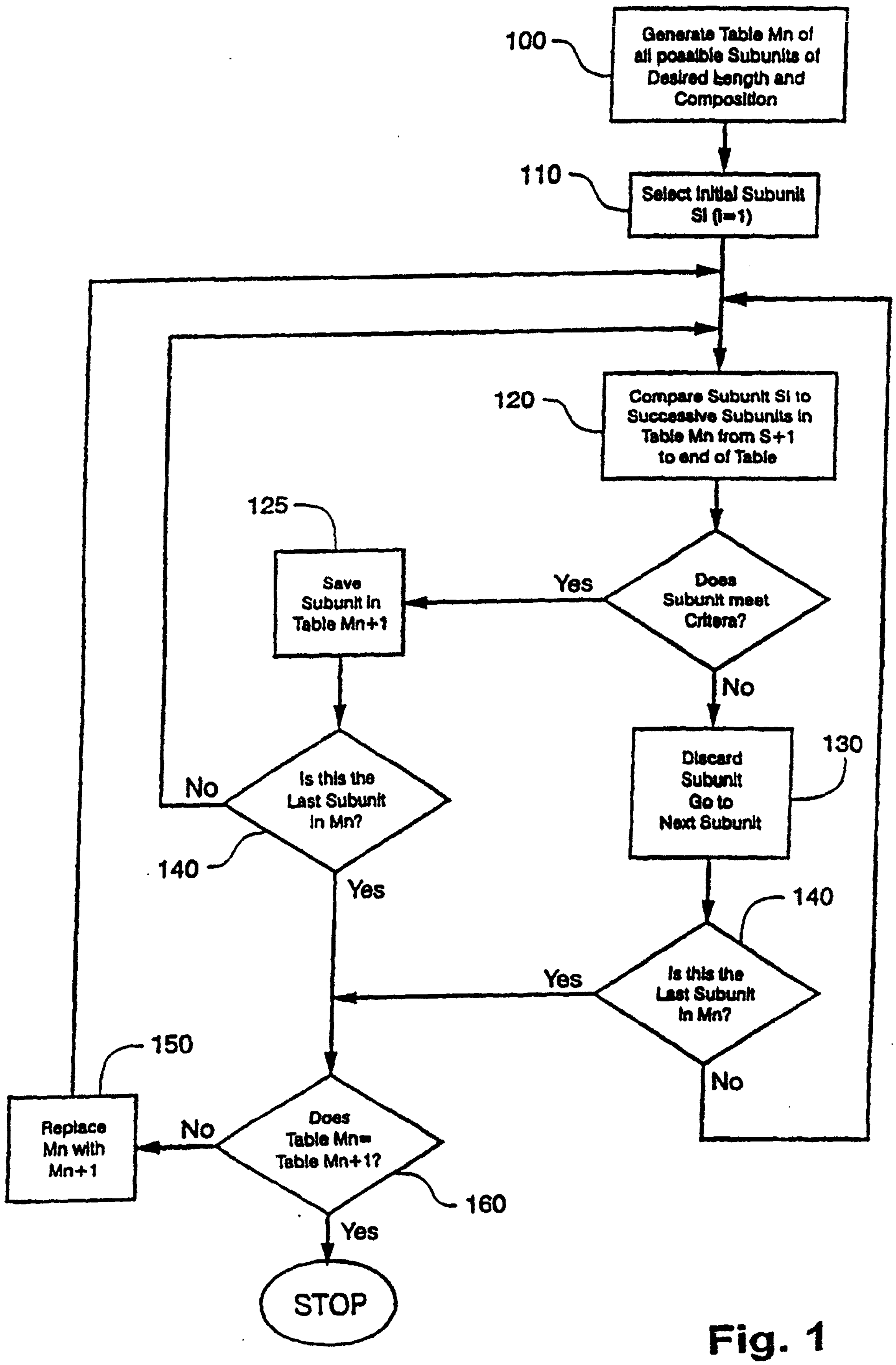
100
Generate Table Mn of all possible Subunits of Desired Length and Composition
110
Select Initial Subunit Si (i=1)
120
Compare Subunit Si to Successive Subunits in Table Mn from S+1 to end of Table
125
Save Subunit in Table Mn+1
Yes
Does Subunit meet Critera?
No
Discard Subunit Go to Next Subunit
130
No
Is this the Last Subunit in Mn?
140
Yes
140
Yes
Is this the Last Subunit in Mn?
No
150
Replace Mn with Mn+1
No
Does Table Mn= Table Mn+1?
160
Yes
STOP

Fig. 1

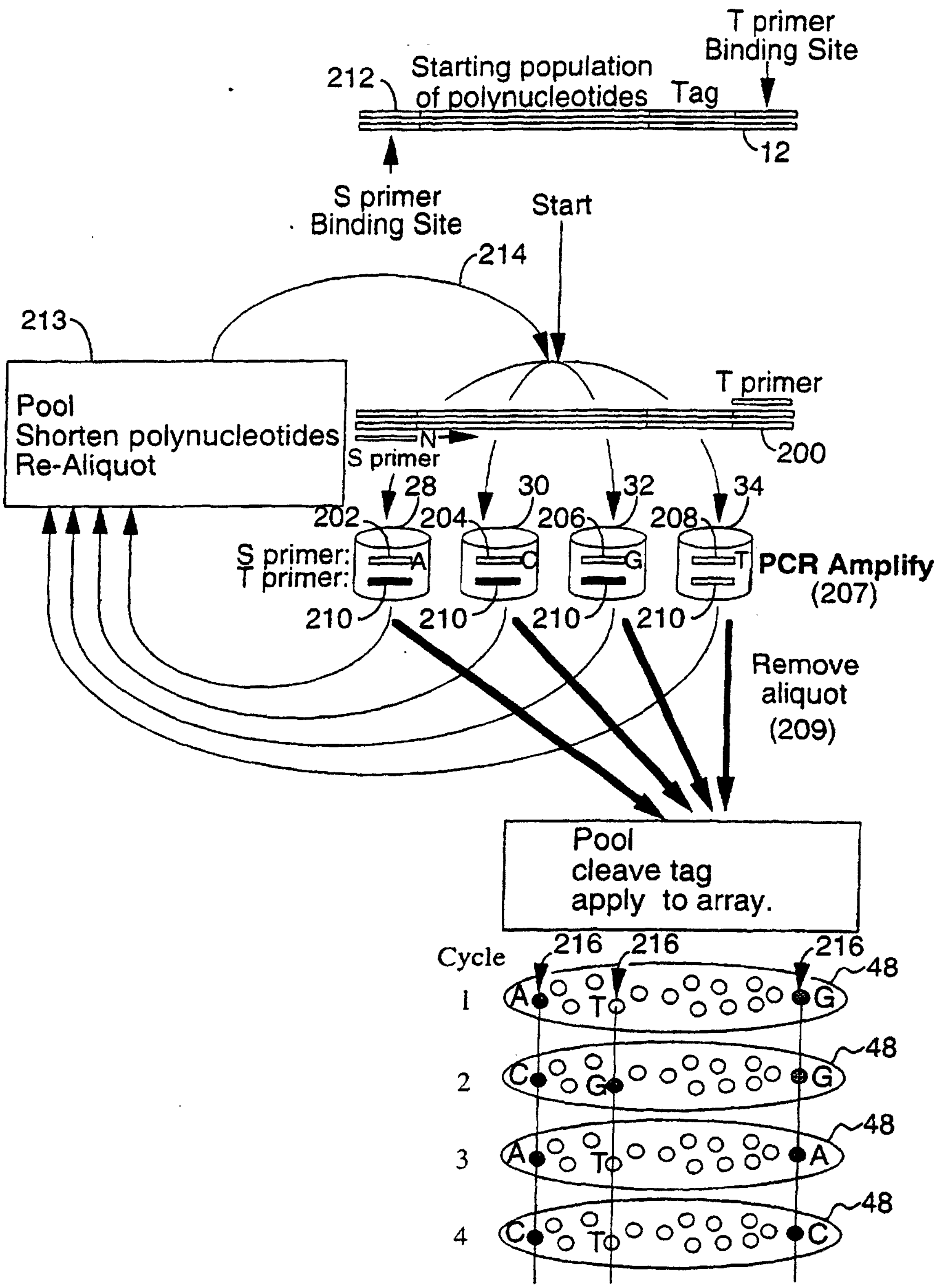
T primer
Binding Site
212
Starting population
of polynucleotides
Tag
12
S primer
Binding Site
Start
214
213
Pool
Shorten polynucleotides
Re-Aliquot
T primer
N
S primer
200
28
30
32
34
202
204
206
208
S primer:
T primer:
A
C
G
T
PCR Amplify
(207)
210
210
210
210
Remove
aliquot
(209)
Pool
cleave tag
apply to array.
Cycle
216
216
216
48
1
A
T
G
2
C
G
G
3
A
T
A
4
C
T
C

Fig. 2a

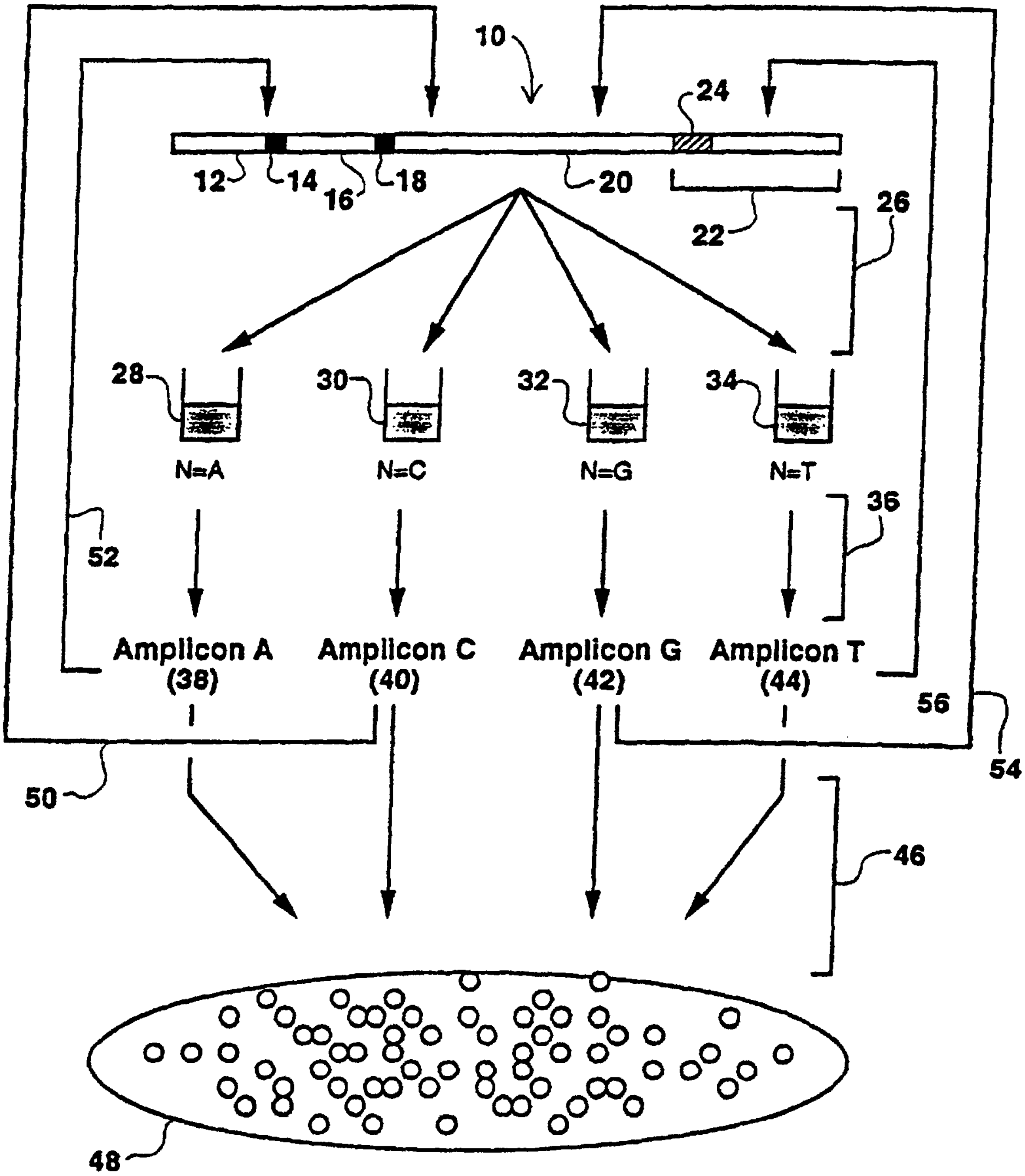
10
24
12
14
16
18
20
22
26
28
30
32
34
N=A
N=C
N=G
N=T
36
52
Amplicon A
(38)
Amplicon C
(40)
Amplicon G
(42)
Amplicon T
(44)
56
54
50
46
48

Fig. 2b

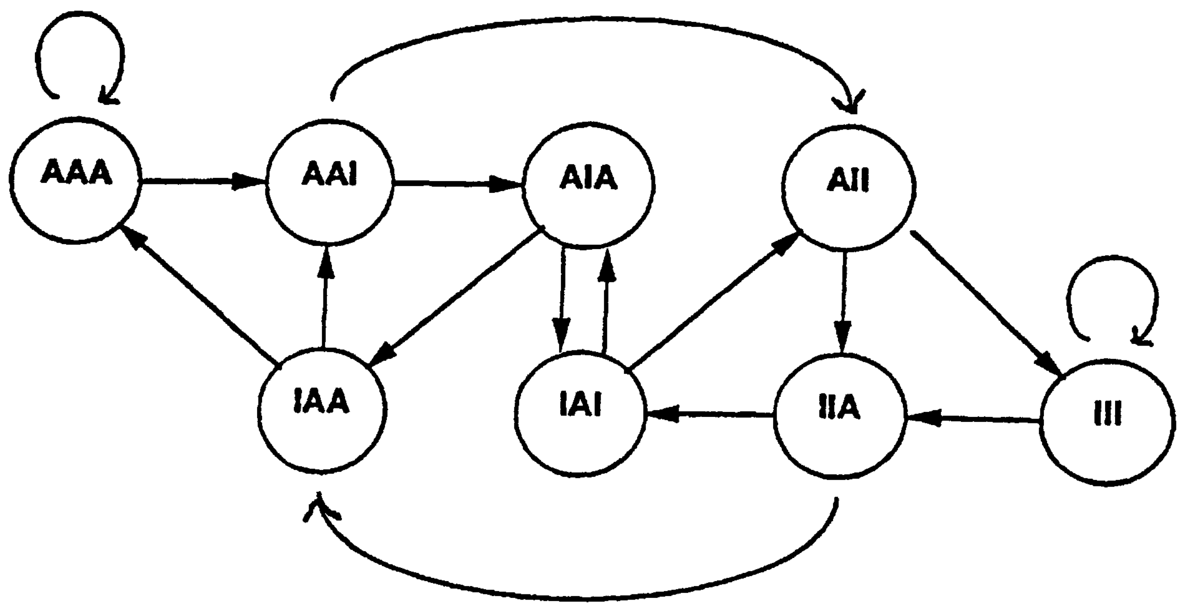


Fig. 2c

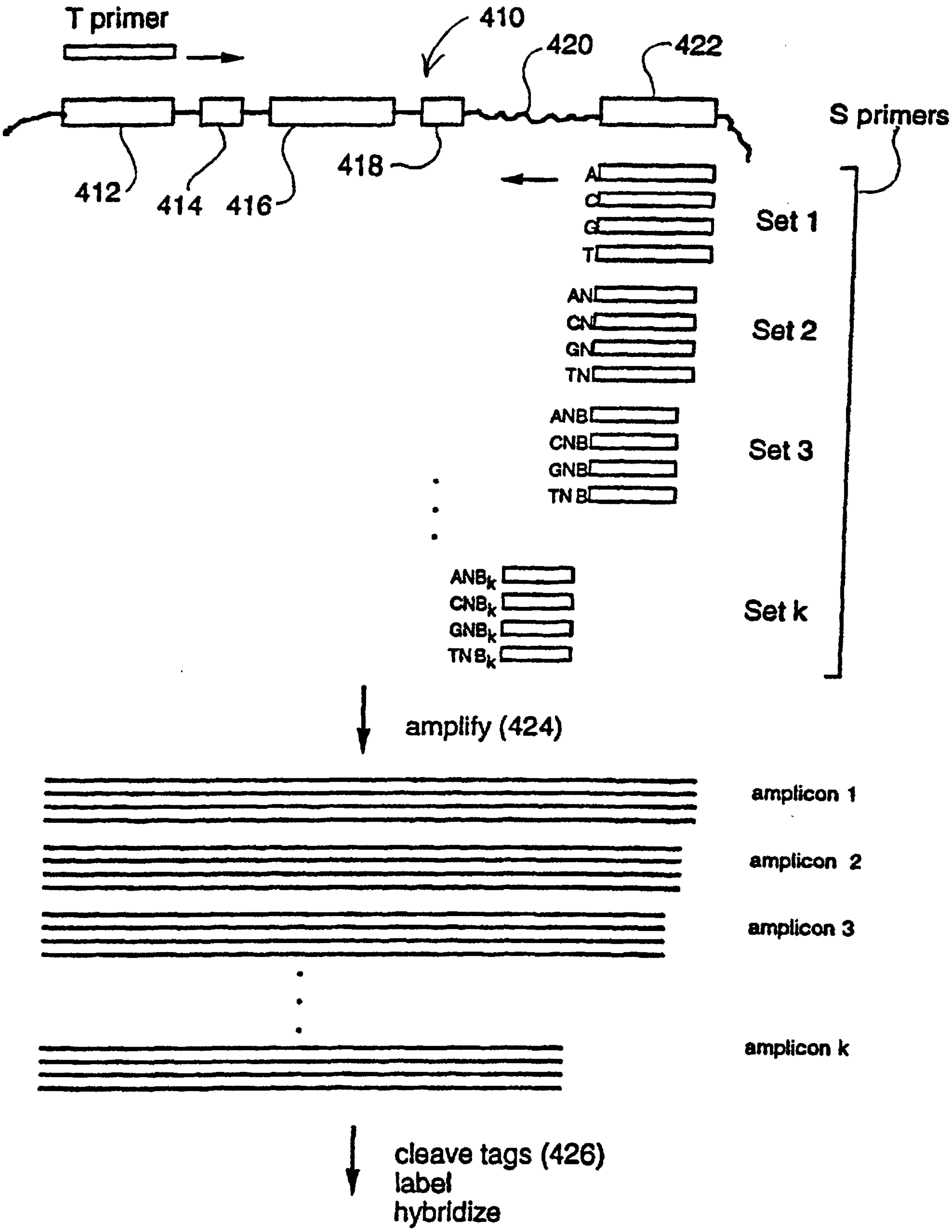
T primer
410
420
422
S primers
412
414
416
418
A
C
G
T
Set 1
AN
CN
GN
TN
Set 2
ANB
CNB
GNB
TN B
Set 3
ANBk
CNBk
GNBk
TN Bk
Set k
amplify (424)
amplicon 1
amplicon 2
amplicon 3
amplicon k
cleave tags (426)
label
hybridize

Fig. 2d

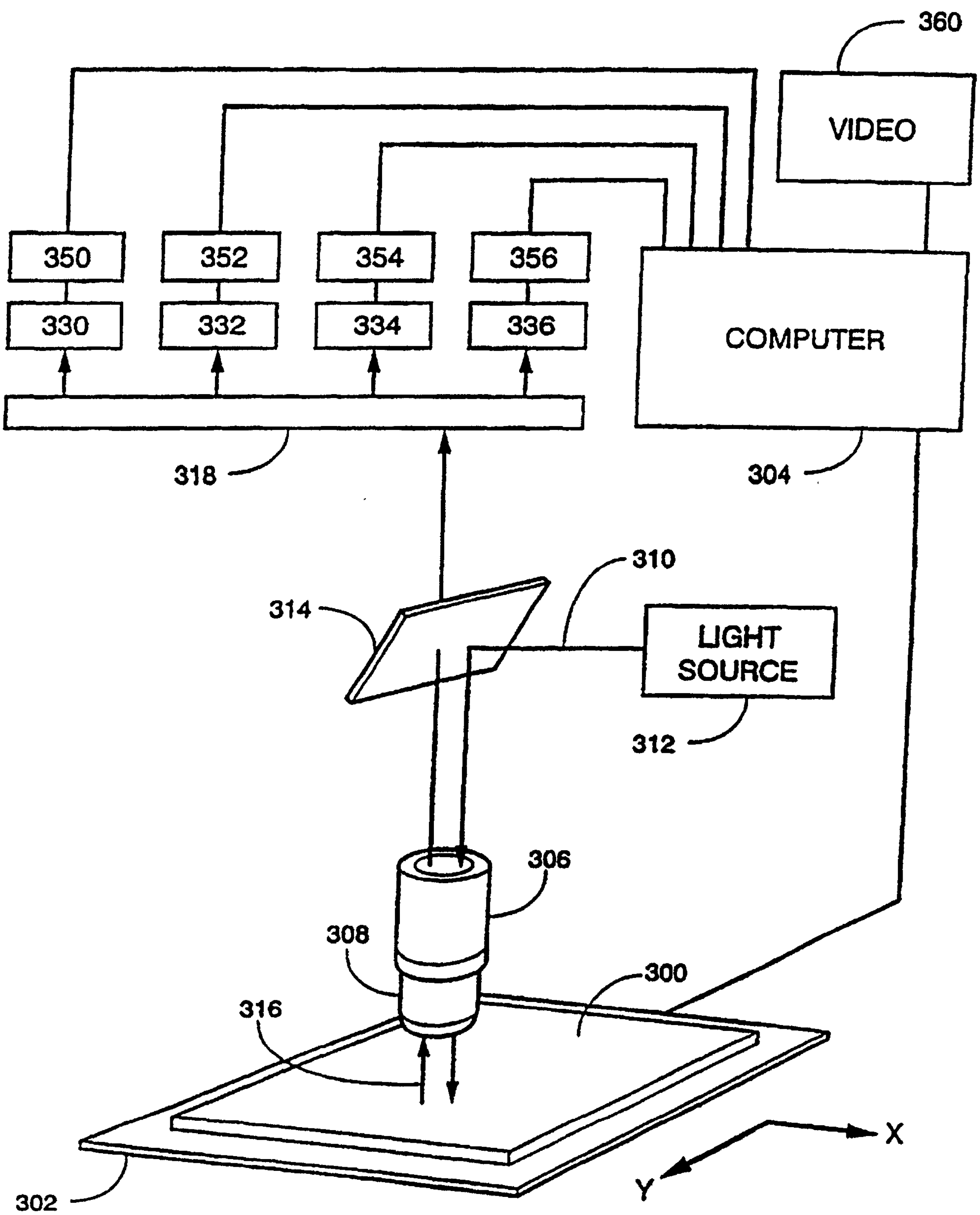
360
VIDEO
350
352
354
356
330
332
334
336
COMPUTER
318
304
310
314
LIGHT SOURCE
312
306
308
300
316
X
Y
302

Fig. 3